# EUROPEAN PATENT APPLICATION

(11) **EP 4 494 569 A1**
(43) Date of publication of application: **22.01.2025**
(21) Application number: 24189260.3
(22) Date of filing: 17.07.2024
(51) Int. Cl.: A61B 8/08, A61B 1/00, A61B 1/313, A61B 8/12, A61B 8/00, A61B 90/00, G16H 30/40, A61B 17/34, A61B 34/20

(54) **MEDICAL SUPPORT DEVICE, OPERATION METHOD OF MEDICAL SUPPORT DEVICE, OPERATION PROGRAM OF MEDICAL SUPPORT DEVICE, AND MEDICAL SUPPORT SYSTEM**

(30) Priority: 21.07.2023 JP 2023119508
(71) Applicant: FUJIFILM Corporation, Tokyo 106-8620 (JP)
(72) Inventor: SASUGA, Saeko, Tokyo (JP); ISHIDA, Koichi, Chiba (JP)
(74) Representative: Dehns Germany Partnerschaft mbB

(57) **Abstract**

A processor of a medical support device is configured to: acquire an intracorporeal image that is captured by an intracorporeal camera that images an inside of a body of a subject, the intracorporeal image including, within an imaging range, an organ punctured by a puncture needle, an insertion part of a medical device inserted into the body, and a marker that is provided at the insertion part and is image-recognizable; derive position/posture information including at least one of a position or a posture of the insertion part based on the marker; and execute display control of displaying a puncture path of the puncture needle in a superimposed form at a position specified in the intracorporeal image based on the position/posture information.

## Description

### BACKGROUND OF THE INVENTION

### 1. Field of the Invention

The technique of the present disclosure relates to a medical support device, an operation method of a medical support device, an operation program of a medical support device, and a medical support system.

### 2. Description of the Related Art

In the endoscopic surgical operation, an ultrasound image of an inside of a liver is acquired by using an ultrasound probe, and a puncture target, which is a position of a tumor, is understood in the ultrasound image. However, even in a case in which the puncture target is displayed in the ultrasound image, the ultrasound image is an image showing an inside of an organ inside the body, and thus it is difficult to know where on a body surface and in which direction a puncture needle should be inserted.

In order to solve such a problem, a technique of supporting the puncture is used. For example, US8688196B discloses a technique of using a magnetic navigation system using a puncture needle having a distal end provided with a magnetic position sensor, to display a position and a posture of a tip of the puncture needle inserted into a body, in real time. EP3136940A discloses a technique in which a laser pointer that applies laser light toward a body surface side along an inclination of a guide groove is used at a distal end of an ultrasound probe provided with a guide groove for guiding insertion of a puncture needle.

### SUMMARY OF THE INVENTION

US8688196B has a problem in that a large-scale apparatus, such as a magnetic navigation system, is required. In EP3136940A, it is necessary to incorporate a light source that emits laser light in a distal end of a medical device such as an ultrasound probe. In order to incorporate the light source, it is necessary to modify an internal structure of the ultrasound probe, and there is a problem that the modification is more time-consuming than in a case of modifying an exterior.

The technique of the present invention provides a medical support device, an operation method of a medical support device, an operation program of a medical support device, and a medical support system that can display a puncture path at an appropriate position in accordance with a position or a posture of a medical device with a simple configuration.

According to an aspect of the present invention, there is provided a medical support device comprising: a processor, in which the processor is configured to: acquire an intracorporeal image that is captured by an intracorporeal camera that images an inside of a body of a subject, the intracorporeal image including, within an imaging range, an organ punctured by a puncture needle, an insertion part of a medical device inserted into the body, and a marker that is provided at the insertion part and is image-recognizable; derive position/posture information including at least one of a position or a posture of the insertion part based on the marker; and execute display control of displaying a puncture path of the puncture needle in a superimposed form at a position specified in the intracorporeal image based on the position/posture information.

It is preferable that the processor is configured to, based on two intracorporeal images that are captured from at least two different viewpoints and are the intracorporeal images in a state in which an operation of causing a change in an interior wall inside the body is performed from an outside of the body and the puncture path is displayed in a superimposed form, determine whether or not a change position at which the interior wall is changed and the puncture path intersect with each other in a three-dimensional space inside the body, and notify of a determination result.

It is preferable that, in a case in which the change position at which the change occurs and the puncture path displayed in a superimposed form intersect with each other two-dimensionally in both of the two intracorporeal images, it is determined that the change position and the puncture path intersect with each other in the three-dimensional space.

It is preferable that the processor is configured to, in a case in which the operation is performed a plurality of times, display a history of the change position of each of the plurality of times of the operations in a superimposed form on the intracorporeal image.

It is preferable that the processor is configured to display the history of the change position by reflecting a movement amount of the viewpoint of the intracorporeal camera in a case in which the viewpoint is moved among the plurality of times of the operations.

It is preferable that the operation is a pressing operation of pressing a body surface of the subject from the outside of the body, and the change position is a protrusion portion in which the interior wall protrudes in a body internal direction via the pressing operation.

It is preferable that the processor is configured to derive an intersection point in a three-dimensional space between an interior wall inside the body and the puncture path based on depth information of the inside of the body indicating a distribution of a depth that is a distance in a depth direction from a viewpoint of the intracorporeal camera.

It is preferable that a camera configured to estimate the depth is used as the intracorporeal camera, and the processor is configured to acquire the depth information.

It is preferable that the processor is configured to acquire the depth information based on two intracorporeal images captured from different viewpoints.

It is preferable that the processor is configured to acquire the depth information by using a machine learning model that receives input of the intracorporeal image and outputs the depth information.

It is preferable that the processor is configured to, in a case in which the intracorporeal image in a state in which an operation of causing a change in an interior wall of a body cavity of the subject is performed from an outside of the body is acquired, determine whether or not a change position at which the interior wall is changed and the puncture path intersect with each other in the three-dimensional space inside the body based on the depth information, and notify of a determination result.

It is preferable that the medical device is a medical probe configured to observe an internal structure of the organ.

It is preferable that the medical probe is an ultrasound probe.

It is preferable that the medical probe includes a guide groove provided at the insertion part and engaging with the puncture needle to guide insertion of the puncture needle to a target position inside the organ, and the processor is configured to specify the position in the intracorporeal image at which the puncture path is superimposed, based on a relative positional relationship between the marker and the guide groove.

It is preferable that the processor is configured to specify the position in the intracorporeal image at which the puncture path is superimposed, based on a target position inside the organ specified in an internal image of the organ acquired by the medical probe, and a correlation between a coordinate system of the internal image and a coordinate system of the intracorporeal image, the correlation being derived based on the position/posture information.

It is preferable that the processor is configured to, in a case in which the puncture path is set by a preoperative simulation in a three-dimensional image of the organ acquired in advance before a surgical operation, specify the position in the intracorporeal image at which the puncture path is superimposed, by using a correlation between a coordinate system of an internal image of the organ acquired by the medical probe and a coordinate system of the three-dimensional image, and a correlation between the coordinate system of the internal image and a coordinate system of the intracorporeal image, the correlation being derived based on the position/posture information.

It is preferable that the processor is configured to change display of a part of the puncture path.

It is preferable that the part of the puncture path is a part on an interior wall side inside the body, and the processor is configured to highlight the part on the interior wall side.

It is preferable that the part of the puncture path is a part on an organ side, and the processor is configured to make visibility lower in the part on the organ side than in a part on an interior wall side.

It is preferable that the processor is configured to detect an insertion length of the insertion part of the medical device, and decide a range in which the display is changed, based on the detected insertion length.

It is preferable that the processor is configured to decide a range in which the display is changed, based on designation of a user.

It is preferable that the processor is configured to display a plurality of different puncture paths.

According to another aspect of the present invention, there is provided an operation method of a medical support device including a processor, the operation method comprising: via the processor, acquiring an intracorporeal image that is captured by an intracorporeal camera that images an inside of a body of a subject, the intracorporeal image including, within an imaging range, an organ punctured by a puncture needle, an insertion part of a medical device inserted into the body, and a marker that is provided at the insertion part and is image-recognizable; deriving position/posture information including at least one of a position or a posture of the insertion part based on the marker; and executing display control of displaying a puncture path of the puncture needle in a superimposed form at a position specified in the intracorporeal image based on the position/posture information.

According to another aspect of the present invention, there is provided a computer-readable storage medium storing an operation program of a medical support device, the operation program causing a computer to function as the medical support device and causing the computer to execute: a step of acquiring an intracorporeal image that is captured by an intracorporeal camera that images an inside of a body of a subject, the intracorporeal image including, within an imaging range, an organ punctured by a puncture needle, an insertion part of a medical device inserted into the body, and a marker that is provided at the insertion part and is image-recognizable; a step of deriving position/posture information including at least one of a position or a posture of the insertion part based on the marker; and a step of executing display control of displaying a puncture path of the puncture needle in a superimposed form at a position specified in the intracorporeal image based on the position/posture information.

According to another aspect of the present invention, there is provided a medical support system comprising: a medical device that includes an insertion part to be inserted into an inside of a body of a subject and has a marker that is provided at the insertion part and is image-recognizable; an intracorporeal camera that images an intracorporeal image including, within an imaging range, the insertion part, the marker, and an organ punctured by a puncture needle; and a medical support device including a processor, in which the processor is configured to: acquire the intracorporeal image; derive position/posture information including at least one of a position or a posture of the insertion part based on the marker; and execute display control of displaying a puncture path of the puncture needle in a superimposed form at a position specified in the intracorporeal image based on the position/posture information.

According to the technique of the present disclosure, it is possible to display the puncture path at an appropriate position in accordance with the position or the posture of the medical device with a simple configuration.

### BRIEF DESCRIPTION OF THE DRAWINGS

Fig. 1 is a diagram showing an outline of a medical support system including a medical support device.
Fig. 2 is a diagram showing a state of an inside of a body in an endoscopic surgical operation.
Fig. 3 is an explanatory diagram of a first guide groove of an ultrasound probe.
Fig. 4 is an explanatory diagram of a second guide groove of the ultrasound probe.
Fig. 5 is a diagram showing an insertion state of a puncture needle guided by the first guide groove.
Fig. 6 is a diagram showing an insertion state of the puncture needle guided by the second guide groove.
Fig. 7 is a diagram showing a hardware configuration of the medical support device.
Fig. 8 is a flowchart showing a procedure of overall medical support processing.
Fig. 9 is a diagram showing a method of deriving position/posture information of an ultrasound probe based on a marker.
Fig. 10 is a diagram showing a relationship in a position and a posture between the marker and the ultrasound probe.
Fig. 11 is another diagram showing the relationship in the position and the posture between the marker and the ultrasound probe.
Fig. 12 is a diagram showing a method of displaying a puncture path in a superimposed form based on position/posture information.
Fig. 13 is a diagram showing a pressing operation via a finger FG according to a second embodiment.
Fig. 14 is a diagram showing a state in which an insertion position NP is searched for by the pressing operation.
Fig. 15 is a flowchart showing an overall procedure of medical support processing according to the second embodiment.
Fig. 16 is a flowchart showing an example of a procedure of a three-dimensional intersection determination.
Fig. 17 is a diagram showing a state in which a protrusion portion and a first puncture line intersect with each other in both of two operative field images.
Fig. 18 is a diagram showing a state in which the protrusion portion and the first puncture line intersect with each other only in one of the two operative field images.
Fig. 19 is a flowchart showing another example of the procedure of the three-dimensional intersection determination.
Fig. 20 is a diagram showing an example of displaying an operation history of the pressing operation.
Fig. 21 is a diagram showing an example in which an operation history is made to follow a change in a viewpoint of a camera.
Fig. 22 is a diagram showing processing of a processor in the example shown in Fig. 21.
Fig. 23 is a diagram showing an example in which the three-dimensional intersection determination can be performed without image analysis.
Fig. 24 is a flowchart showing an overall procedure of medical support processing according to a third embodiment.
Fig. 25 is a diagram showing an example of a method of acquiring a depth map.
Fig. 26 is a diagram showing an example in which an intersection point is derived from the depth map.
Fig. 27 is a diagram showing a modification example of the method of acquiring the depth map.
Fig. 28 is a diagram showing another modification example of the method of acquiring the depth map.
Fig. 29 is a flowchart showing a processing procedure of an example in which the three-dimensional intersection determination between the first puncture line and the protrusion portion is performed based on the depth map.
Fig. 30 is a diagram showing an example of processing of the three-dimensional intersection determination between the first puncture line and the protrusion portion based on the depth map.
Fig. 31 is a diagram showing an example in which a part of the first puncture line on an interior wall side is highlighted.
Fig. 32 is a diagram showing an example in which visibility of a part of the first puncture line on an organ side is reduced.
Fig. 33 is a diagram showing an example in which a scale is displayed on a part of the first puncture line.
Fig. 34 is a diagram showing an example in which a range in which the display of the first puncture line is changed is decided based on an insertion length of the ultrasound probe.
Fig. 35 is a diagram showing an example in which a plurality of first puncture lines are displayed.
Fig. 36 is a diagram showing a fourth embodiment.
Fig. 37 is a diagram showing a procedure following Fig. 36.
Fig. 38 is a diagram showing a method of creating a preoperative 3D image.
Fig. 39 is a diagram showing a method of creating preoperative preparation information.
Fig. 40 is a diagram showing a method of creating an ultrasound 3D image.
Fig. 41 is a diagram showing a method of using a correlation between the preoperative 3D image and the ultrasound 3D image.

### DESCRIPTION OF THE PREFERRED EMBODIMENTS

### First embodiment

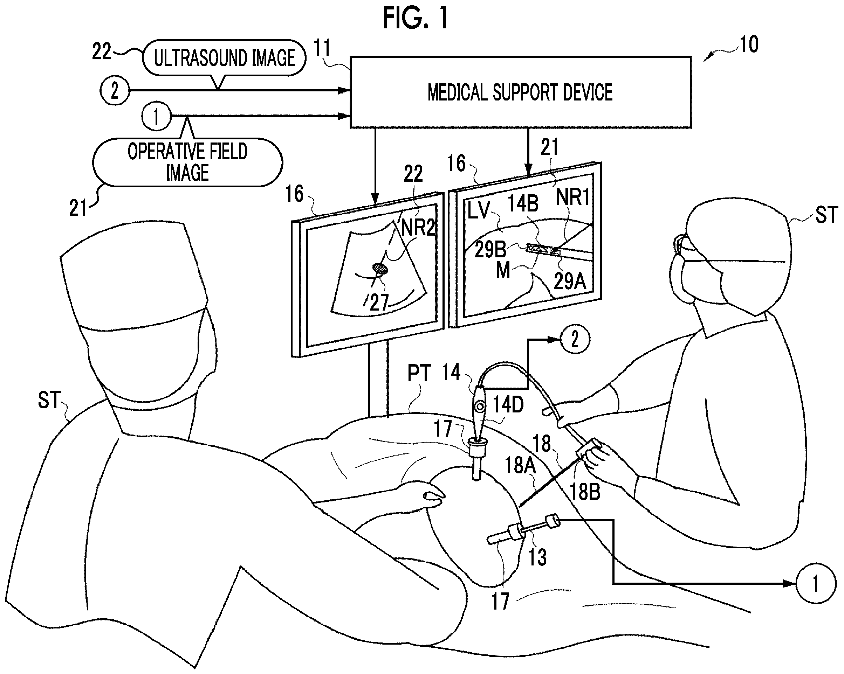

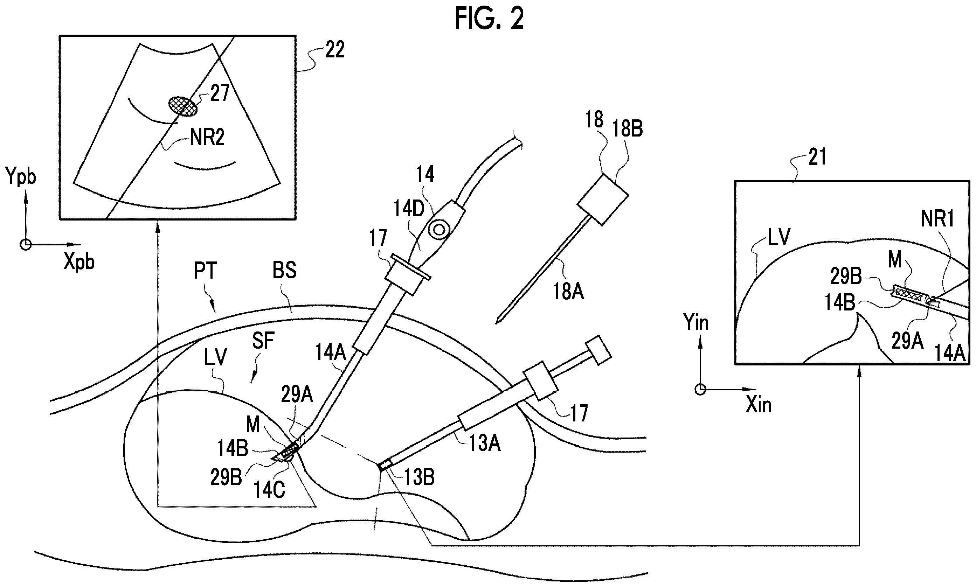

As shown in Figs. 1 and 2, a medical support system 10 is used, for example, in a case in which an endoscopic surgical operation using an endoscope 13 is performed on a patient PT. The endoscopic surgical operation is a surgical operation that is performed by making a small hole in a body of the patient PT and inserting a medical device, such as the endoscope 13, from the hole, unlike a laparotomy. The medical support system 10 provides a medical staff ST including a doctor with a visual field of an operative field inside the body of the patient PT, and with support information for supporting medical care such as a surgical operation and an examination. As will be described below, the support information is, specifically, a puncture path of a puncture needle 18. Since the medical support system 10 has a function of providing the support information in real time during the surgical operation, the medical support system 10 is also called a surgical operation navigation system or the like. The medical support system 10 comprises, for example, a medical support device 11, the endoscope 13, an ultrasound probe 14, and a display 16. The medical support device 11 is an example of a "medical support device" according to the technique of the present disclosure, and the patient PT is an example of a "subject".

The medical support device 11 is communicably connected to the endoscope 13, the ultrasound probe 14, and the display 16. In the endoscopic surgical operation, a part of the endoscope 13 and a part of the ultrasound probe 14 including distal end parts thereof are inserted into the body through a trocar 17. The trocar 17 is an insertion tool having an insertion hole into which the endoscope 13 or the like is inserted and a valve provided in the insertion hole to prevent gas leakage. In the endoscopic surgical operation, since an abdominal cavity is inflated with air by injecting carbon dioxide gas, the trocar 17 is used to insert the endoscope 13, the ultrasound probe 14, and the like into the body.

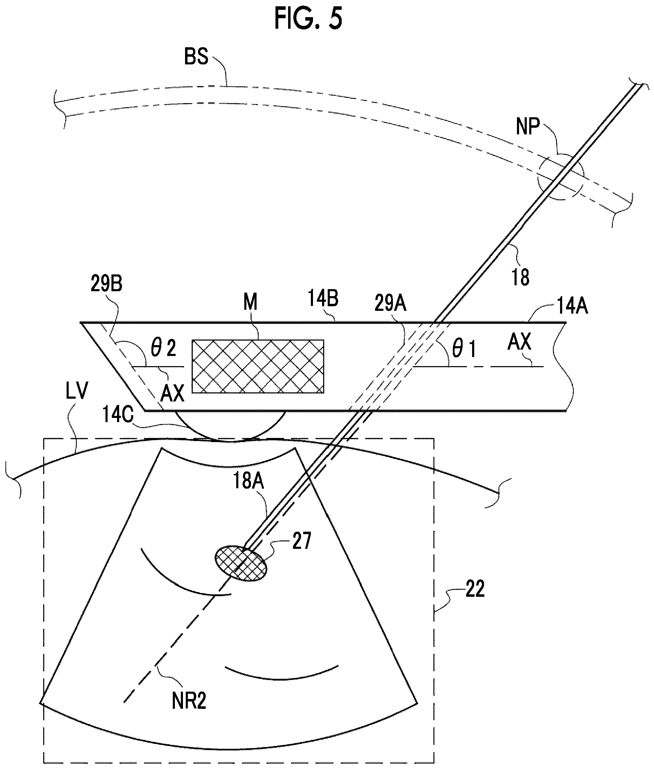

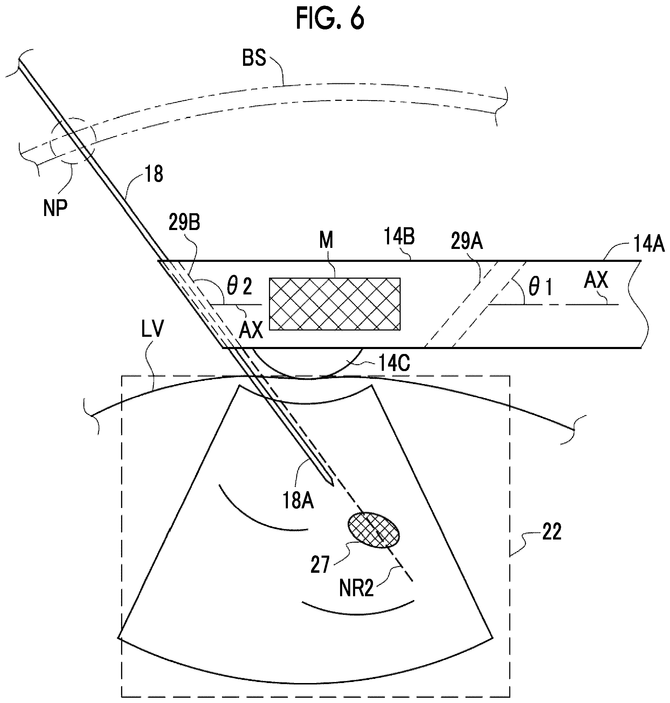

In the example, a target part in the surgical operation is a liver LU, and Fig. 2 shows a state in which the endoscope 13 and the ultrasound probe 14 are inserted into the abdomen of the patient PT. The puncture needle 18 is a treatment tool for performing the puncture using, as a puncture target, a lesion such as a tumor included in an organ as a puncture target. The puncture needle 18 is, for example, a cauterization puncture needle used for cauterization of the lesion. The cauterization puncture needle is provided with an electrode to which a high-frequency voltage is applied at a distal end, and in a case in which the high-frequency voltage is applied in a state in which the lesion is punctured by the electrode, the lesion is necrotized by the heat generated by the electrode. In the present example, as the surgical operation, a case will be described in which a treatment of necrotizing a tumor 27 is performed by visualizing the tumor 27 of the liver LV by using an ultrasound image 22 and cauterizing the visualized tumor 27 (see also Figs. 5 and 6) by using the puncture needle 18. The puncture needle 18 has a needle part 18A and a grip part 18B provided on a base end side of the needle part 18A.

The endoscope 13 has an insertion part 13A to be inserted into the body of the patient PT, and a camera 13B and a light source for illumination (such as a light emitting diode (LED)) are incorporated in a distal end part of the insertion part 13A. The endoscope 13 is, for example, a rigid endoscope in which the insertion part 13A is rigid, and is often used for abdominal cavity observation, so the endoscope 13 is also called a laparoscope. The camera 13B includes an image sensor such as a charge coupled device (CCD) image sensor and a complementary metal oxide semiconductor (CMOS) image sensor, and an imaging optical system including a lens that forms a subject image on an imaging surface of the image sensor. The image sensor is, for example, an image sensor capable of capturing a color image. The camera 13B is an example of an "intracorporeal camera" according to the technique of the present disclosure.

The endoscope 13 optically images an operative field SF including the target part (in the present example, the liver LU) inside the body of the patient PT via the camera 13B. The operative field SF is a space that spreads in a body cavity defined by an organ and a body wall inside the body. The endoscope 13 is connected to an image processing processor for an endoscope (not shown), and the image processing processor performs signal processing on a imaging signal output by the image sensor to generate an operative field image 21 of the operative field SF inside the body. The operative field image 21 is an example of an "intracorporeal image captured by an intracorporeal camera" according to the technique of the present disclosure.

As the illumination light of the endoscope 13, in some cases, special light such as ultraviolet rays and infrared light is used, but visible light such as white light is also used. It should be noted that, as the illumination light of the endoscope 13, the special light such as the ultraviolet rays and the infrared light may be used. As the special light, light limited to a specific wavelength, such as short-wavelength narrow-band light obtained by narrowing light in a short wavelength range such as an ultraviolet range may be used. The operative field image 21 captured by the endoscope 13 is transmitted to the medical support device 11 in real time via the image processing processor for the endoscope. In Fig. 2, reference numerals Xin and Yin indicate a coordinate system of the operative field image 21.

As the support information for guiding the insertion of the puncture needle 18, a first puncture line NR1 indicating a puncture path of the puncture needle 18 is displayed in a superimposed form on the operative field image 21. The first puncture line NR1 will be described below.

The ultrasound probe 14 has an insertion part 14A to be inserted into the body of the patient PT, and an operating part 14D on a base end side of the insertion part 14A, similarly to the endoscope 13. An ultrasound transducer 14C is incorporated in a distal end part 14B of the insertion part 14A. The ultrasound transducer 14C transmits the ultrasound wave to the target part and receives the reflected wave reflected by the target part. The ultrasound probe 14 is connected to an image processing processor for an ultrasound probe (not shown). The image processing processor for the ultrasound probe performs image reconstruction processing based on the reflected wave based on a signal corresponding to the reflected wave received by the ultrasound probe 14. The image reconstruction processing generates the ultrasound image 22 showing an internal structure of the target part scanned by the ultrasound probe 14. The ultrasound image 22 is a so-called brightness (B)-mode image in which the internal structure from a surface layer to a deep layer to which the ultrasound wave reaches the target part is visualized as brightness information. The ultrasound image 22 visualizes the internal structure of the target part that cannot be observed in the operative field image 21 obtained by optical imaging. The ultrasound probe 14 is an example of a "medical device" and a "medical probe" according to the technique of the present disclosure.

As the support information for guiding the insertion of the puncture needle 18, a second puncture line NR2 indicating a puncture path of the puncture needle 18 is displayed in a superimposed form on the ultrasound image 22. The second puncture line NR2 will be described below.

The ultrasound probe 14 is, for example, a convex type that radially transmits ultrasound waves, and acquires a fan-shaped image with the ultrasound transducer 14C as a base point. A plurality of ultrasound images 22 are captured along a scanning direction by performing the scanning with the ultrasound probe 14. The ultrasound image 22 is transmitted to the medical support device 11 in real time through the image processing processor for the ultrasound probe. In Fig. 2, reference numerals Xpb and Ypb indicate a coordinate system of the ultrasound image 22.

In the ultrasound probe 14, a guide groove 29 is provided at the distal end part 14B of the insertion part 14A. The guide groove 29 is a guide groove for engaging with the puncture needle 18 to guide the insertion of the puncture needle 18 to the target position inside the organ. The guide groove 29 includes two guide grooves of a first guide groove 29A and a second guide groove 29B. The first guide groove 29Ais the guide groove 29 provided at the distal end part 14B on the base end side with respect to the ultrasound transducer 14C. The second guide groove 29B is the guide groove 29 formed at the most distal end of the distal end part 14B and provided on the distal end side with respect to the ultrasound transducer 14C. Hereinafter, in a case in which it is not necessary to distinguish between the first guide groove 29A and the second guide groove 29B, the first guide groove 29A and the second guide groove 29B are collectively referred to as the guide groove 29.

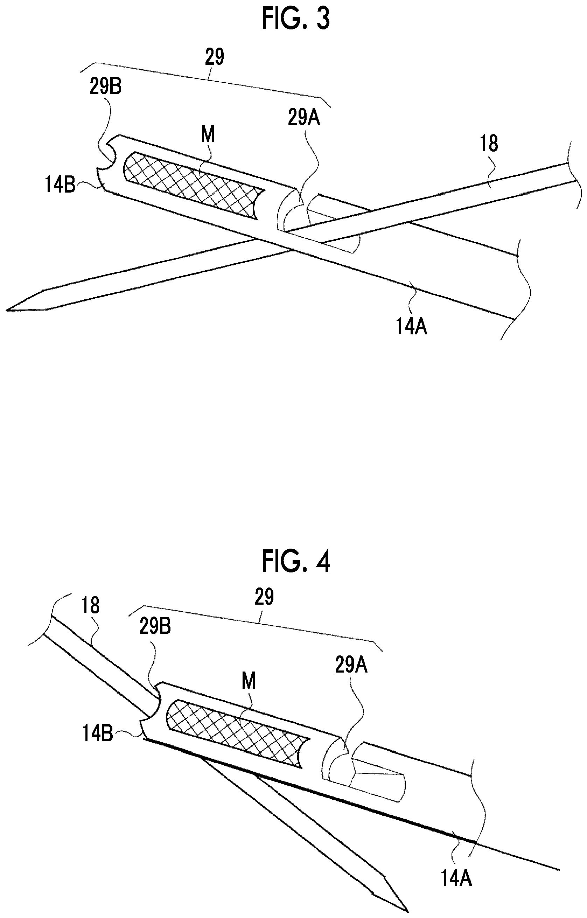

Fig. 3 shows a state of the puncture needle 18 guided by the first guide groove 29A, and Fig. 4 shows a state of the puncture needle 18 guided by the second guide groove 29B. Fig. 5 schematically shows a state in which the puncture needle 18 inserted into the body is guided by the first guide groove 29A, and the tumor 27 in the liver LV is punctured by the puncture needle 18. Fig. 6 schematically shows a state in which the puncture needle 18 inserted into the body is guided by the first guide groove 29A and the tumor 27 in the liver LV is punctured by the puncture needle 18.

The first guide groove 29Ais inclined at an angle of θ1 with respect to a direction of an axis AX of the distal end part 14B. The first guide groove 29A is inclined rearward such that a needle tip of the puncture needle 18 to be inserted from the base end side of the distal end part 14B is directed toward the distal end side of the distal end part 14B. On the contrary, the second guide groove 29B is inclined forward such that the needle tip of the puncture needle 18 to be inserted from the distal end side of the distal end part 14B is directed toward the base end side of the distal end part 14B.

The puncture needle 18 is inserted while the tumor 27 is checked by the ultrasound image 22. In a case in which both the first guide groove 29A and the second guide groove 29B are inclined toward the ultrasound transducer 14C, the needle tip of the puncture needle 18 can be directed toward a region visualized by the ultrasound image 22.

The distal end part 14B is provided with a marker M. The marker M is a marker that is recognizable in an optically captured image, that is, an optically detectable marker. The marker M can be imaged by the camera 13B of the endoscope 13. The medical support device 11 estimates a position and a posture of the guide groove 29 provided at the distal end part 14B by using the marker M. As will be described below, specifically, a position and a posture of the distal end part 14B of the insertion part 14A are estimated by the marker M. The posture of the distal end part 14B is represented by, for example, the direction of the axis AX of the distal end part 14B. Then, the position and the posture of the guide groove 29 of which a relative positional relationship with the distal end part 14B is known are estimated based on the estimated direction of the axis AX of the distal end part 14B.

For example, the marker M is formed of a pattern having a morphological feature such as a lattice pattern or a dot pattern. The method of estimating the position and the posture using the marker M will be described below. The marker M is an example of a "marker that is image-recognizable" according to the technique of the present disclosure. The insertion part 14A is an example of an "insertion part of a medical device" according to the technique of the present disclosure.

The medical support device 11 acquires the operative field image 21 from the endoscope 13, and acquires the ultrasound image 22 from the ultrasound probe 14. As shown in Fig. 1, two displays 16 are provided as an example, and two images of the operative field image 21 and the ultrasound image 22 are displayed on the respective displays 16. The operative field image 21 and the ultrasound image 22 are output as a video, and are displayed on the display 16 as a live view.

In a case in which the ultrasound probe 14 is inserted into the operative field SF, the ultrasound probe 14 is shown in the operative field image 21. The medical support device 11 outputs, to the display 16, the operative field image 21 in which the insertion part 14A (including the distal end part 14B and the marker M) of the ultrasound probe 14 is shown. The visual field of the operative field SF inside the body of the patient PT is provided to the medical staff ST through a screen of the display 16.

As shown in Figs. 5 and 6, it is possible to display two types of the second puncture lines NR2, which respectively indicate the puncture path along the first guide groove 29A at the inclination angle θ1 and the puncture path along the second guide groove 29B at the inclination angle θ2, in a superimposed form on the ultrasound image 22. The second puncture line NR2 is the support information for guiding the puncture with the puncture needle 18 to the target position inside the organ in the ultrasound image 22.

The second puncture line NR2 is information indicating a path passing through the first guide groove 29A or the second guide groove 29B for guiding the puncture needle 18. The second puncture line NR2 is generated as an extension line of the guide groove 29 based on a known positional relationship between the ultrasound transducer 14C and the guide groove 29. The second puncture line NR2 is used as a guide in a case in which the target position is punctured by the puncture needle 18. For example, in a case in which the target position punctured by the puncture needle 18 is the tumor 27 in the liver LV, the positioning of the ultrasound probe 14 is performed by the medical staff ST such that the second puncture line NR2 and the tumor 27 overlap in the ultrasound image 22. In this state, the tumor 27 is punctured by the puncture needle 18 with the second puncture line NR2 as a guide. In this way, in a state in which the position of the ultrasound probe 14 is adjusted such that the second puncture line NR2 passes through the target position, the puncture needle 18 can reach the target position by passing the puncture needle 18 through the guide groove 29.

However, since the ultrasound image 22 is the internal image of the organ, in a case in which only the second puncture line NR2 is displayed in a superimposed form on the ultrasound image 22, it is difficult to understand in which position and in what posture on a body surface BS the puncture needle 18 should be inserted in a case of inserting the puncture needle 18 into the body from the outside of the body. Specifically, as shown in Figs. 5 and 6, it is difficult to understand an insertion angle of the puncture needle 18 from the body surface BS toward the organ and an insertion position NP of the puncture needle 18 on the body surface BS of the patient PT. Therefore, the medical support device 11 displays the first puncture line NR1 of the puncture needle 18 in a superimposed form in the operative field image 21. As a result, the medical staff ST is provided with the support information serving as a guide for the insertion angle and the insertion position NP of the puncture needle 18. The first puncture line NR1 is an example of a "puncture path of the puncture needle" according to the technique of the present disclosure.

As described above, the medical support device 11 displays the first puncture line NR1 in a superimposed form on the operative field image 21 in addition to displaying the second puncture line NR2 in a superimposed form on the ultrasound image 22 as the support information for supporting the puncture of the target position with the puncture needle 18. The first puncture line NR1 indicates the puncture path from the body surface BS to the guide groove 29 of the ultrasound probe 14 in the operative field image 21, and the second puncture line NR2 indicates the puncture path from the guide groove 29 to the target position in the organ, such as the tumor 27 of the liver LV, in the ultrasound image 22.

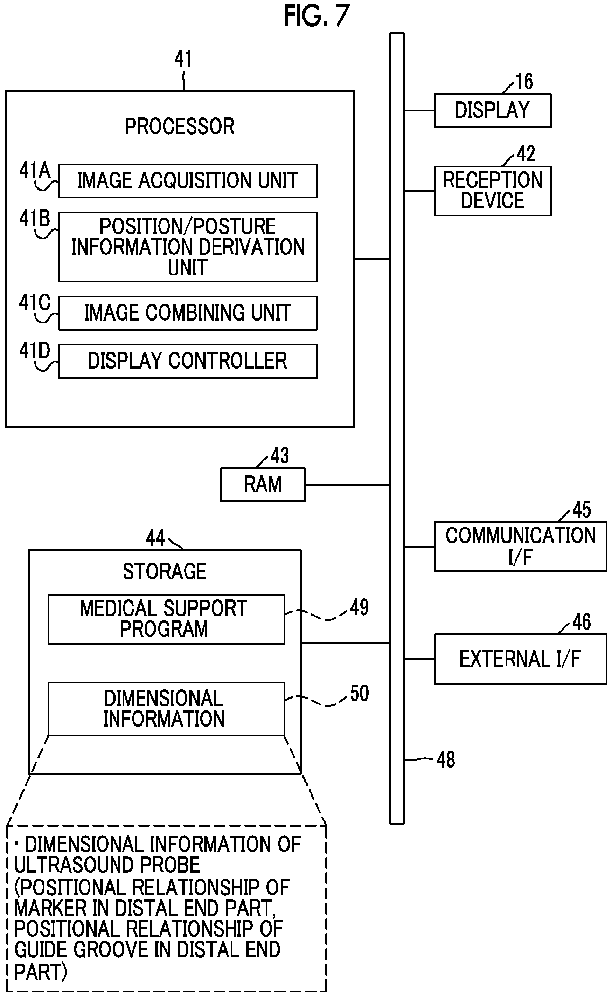

Fig. 7 shows an example of a hardware configuration of the medical support device 11. The medical support device 11 is an example of a "medical support device" and a "computer" according to the technique of the present disclosure, and comprises a processor 41, a reception device 42, the display 16, a random access memory (RAM) 43, a storage 44, a communication I/F 45, and an external I/F 46. The respective units are connected to a bus 48 and can communicate with each other.

The medical support device 11 is operated by an operator, such as the medical staff ST, through the reception device 42. The reception device 42 includes a keyboard, a mouse, and the like (not shown), and receives an instruction from the operator. The reception device 42 may be a device that receives touch input, such as a touch panel, a device that receives voice input, such as a microphone, a device that receives gesture input, such as a camera, or the like.

Examples of the display 16 include an electro-luminescence (EL) display and a liquid crystal display. As described above, there are two displays 16, and various types of information are displayed on each display 16, in addition to the operative field image 21 and the ultrasound image 22.

The processor 41 is, for example, a central processing unit (CPU), and integrally controls the respective units of the medical support device 11 in accordance with a control program and executes various types of processing in accordance with various types of application programs.

The storage 44 is a nonvolatile storage device that stores various programs, various parameters, and the like. Examples of the storage 44 include a hard disk drive (HDD) and a solid state drive (SSD). The storage 44 stores a medical support program 49 that causes a computer to function as the medical support device 11.

The RAM 43 is a memory that transitorily stores information, and is used as a work memory by the processor 41. Examples of the RAM 43 include a dynamic random access memory (DRAM) or a static random access memory (SRAM).

The communication I/F 45 is connected to a network (not shown), such as a local area network (LAN) and/or a wide area network (WAN), and performs transmission control in accordance with a communication protocol defined in various types of wired or wireless communication standards.

The external I/F 46 is, for example, a universal serial bus (USB) interface, and is used for connection to peripheral devices, such as a printer and a memory card.

The processor 41 executes medical support processing by reading out the medical support program 49 from the storage 44 and executing the medical support program 49 on the RAM 43. The medical support processing is realized by the processor 41 operating as an image acquisition unit 41A, a position/posture information derivation unit 41B, an image combining unit 41C, and a display controller 41D. The medical support program 49 is an example of an "operation program of a medical support device" according to the technique of the present disclosure.

Dimensional information 50 includes dimensional information of the ultrasound probe 14. The dimensional information of the ultrasound probe 14 includes, specifically, information indicating a positional relationship of the marker M in the distal end part 14B of the ultrasound probe 14 and a positional relationship of the guide groove 29 in the distal end part 14B. The positional relationship of the marker M in the distal end part 14B is, for example, information on the position and the posture at which the lattice of the marker M of the lattice pattern is provided with respect to an axial direction and a circumferential direction of the distal end part 14B. The positional relationship of the guide groove 29 in the distal end part 14B is a linear distance in the axial direction along the axis AX of the distal end part 14B between a reference point of the distal end part 14B and the guide groove 29 (including the first guide groove 29A and the second guide groove 29B), an inclination angle θ (including θ1 and θ2) of the guide groove 29 with respect to the axial direction of the distal end part 14B, or the like.

The image acquisition unit 41A executes image acquisition processing of acquiring the operative field image 21 and the ultrasound image 22. For example, the image acquisition unit 41A acquires the operative field image 21 or/and the ultrasound image 22 from the device including the processor of the endoscope 13 or/and the processor of the ultrasound probe 14 through the external I/F 46 or the communication I/F 45. It should be noted that the medical support device 11 may include the processor of the endoscope 13 or/and the processor of the ultrasound probe 14.

The position/posture information derivation unit 41B executes processing of deriving the position/posture information including the position and the posture of the guide groove 29 of the ultrasound probe 14 based on the marker M.

The image combining unit 41C specifies a position for superimposing the first puncture line NR1 in the operative field image 21 based on the derived position/posture information, and superimposes the first puncture line NR1 on the specified position. Here, the position/posture information is an example of "position/posture information including at least one of a position or a posture of the insertion part" according to the technique of the present disclosure. In the present example, the position/posture information includes the position and the posture, but in a case in which the position at which the first puncture line NR1 is superimposed can be specified by any one of the position or the posture, the position/posture information may include information including one of the position or the posture.

The display controller 41D executes control of displaying the operative field image 21 and the ultrasound image 22 on the display 16. In addition, display control of displaying the first puncture line NR1 in a superimposed form on the operative field image 21 is executed. The display control of displaying the first puncture line NR1 in a superimposed form is, specifically, control of generating a composite image in which the first puncture line NR1 is superimposed on the operative field image 21, and displaying the generated composite image on the display 16. Hereinafter, such display control will be simply referred to as display in a superimposed form.

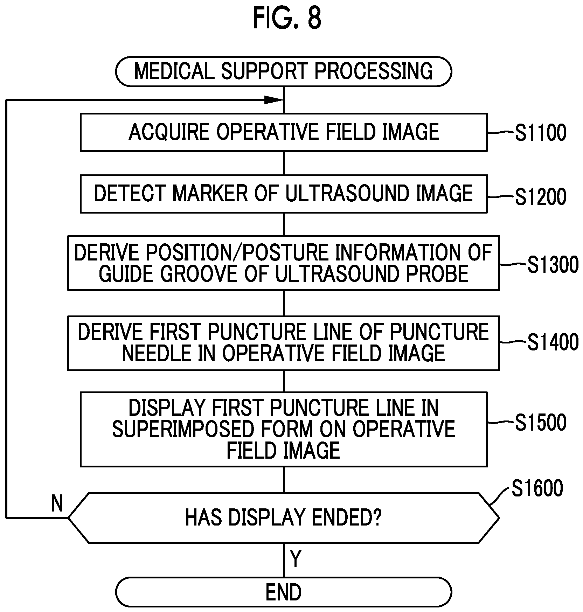

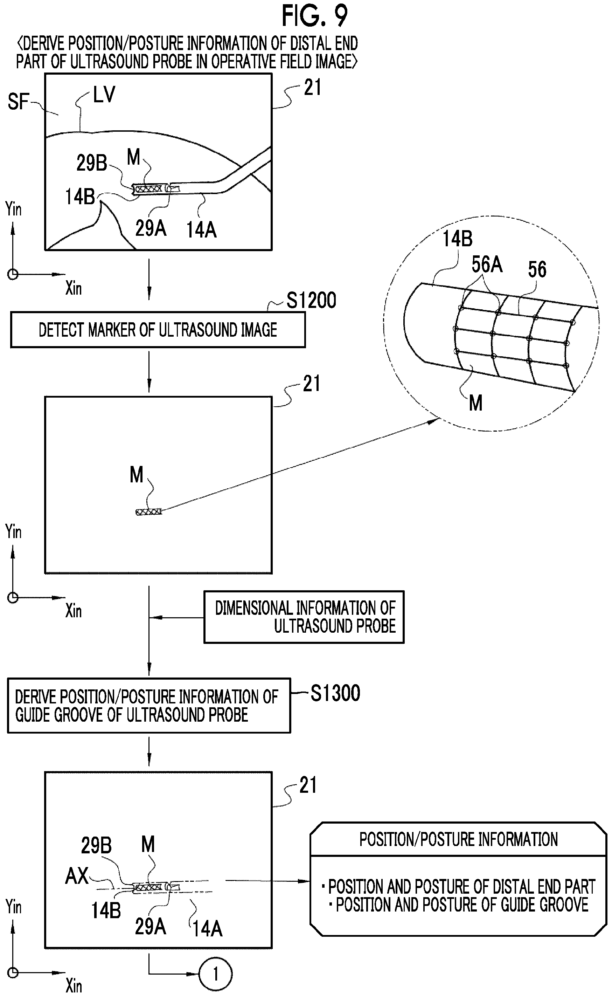

The processing executed by the processor 41 of the medical support device 11 according to the present disclosure will be specifically described with reference to Figs. 8 to 12. Fig. 8 is a flowchart showing an overall processing procedure, and individual processing is described in Figs. 9 to 12.

As shown in Fig. 8, in the medical support processing, in step S 1100, the processor 41 acquires the operative field image 21 captured by the camera 13B of the endoscope 13. In step S1200, the processor 41 detects the marker M of the ultrasound probe 14 from the operative field image 21. In step S1300, the processor 41 derives the position/posture information of the guide groove 29 of the ultrasound probe 14 based on the marker M. In step S1400, the processor 41 derives the first puncture line NR1 of the puncture needle 18 in the operative field image 21 based on the position/posture information of the guide groove 29. In step S1500, the processor 41 displays the first puncture line NR1 in a superimposed form on the operative field image 21. The processor 41 repeats such processing until a display end instruction is input (step S1600).

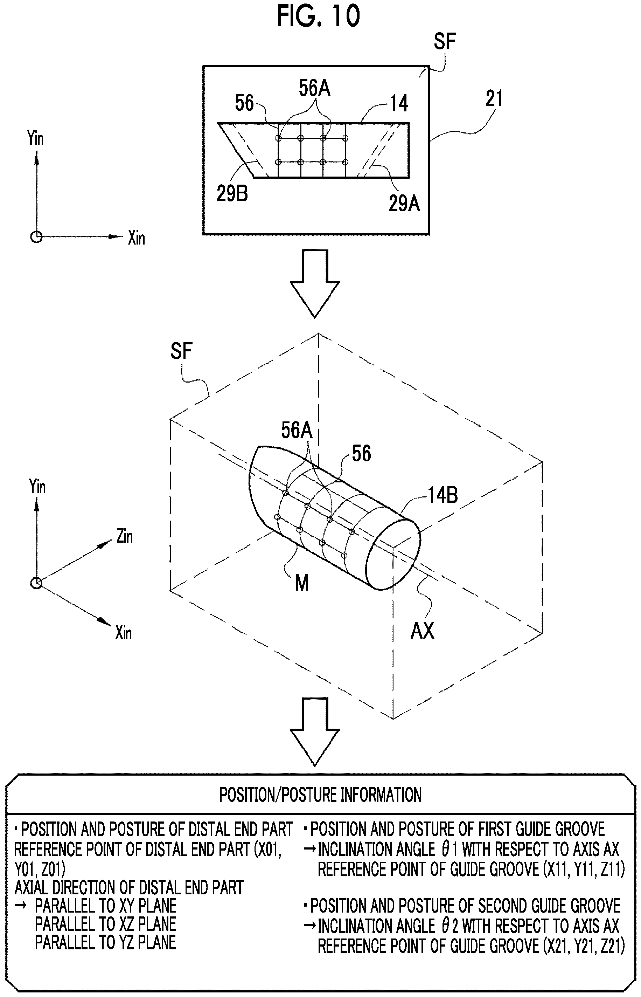

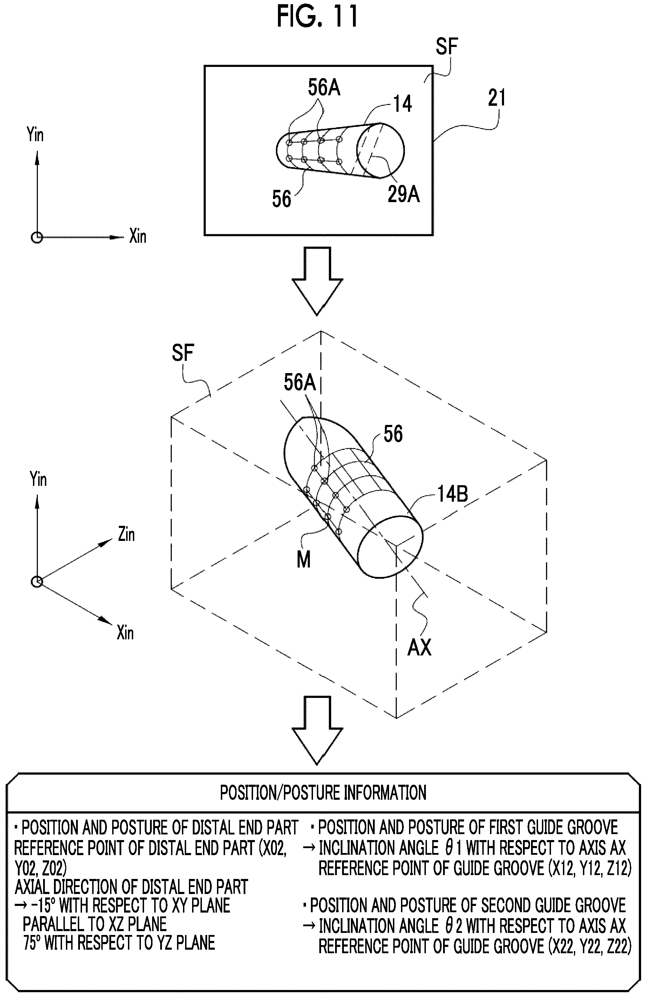

Hereinafter, each processing step will be described in more detail. First, Fig. 9 is a diagram conceptually showing the detection of the marker M in step S1200 shown in Fig. 8 and the derivation of the position/posture information of the guide groove 29 of the ultrasound probe 14 in step S1300. As a supplementary description of Fig. 9, the description will be made with reference to Fig. 10 and Fig. 11 as appropriate.

As shown in Fig. 9, in step S1200, the processor 41 detects the marker M based on the operative field image 21 by using, for example, an image processing method such as pattern matching. The marker M is, for example, a marker of a lattice pattern 56 composed of a first line extending in the direction of the axis AX of the distal end part 14B of the ultrasound probe 14 and a second line orthogonal to the axial direction of the distal end part 14B and formed in the circumferential direction along an outer peripheral surface of the distal end part 14B. A lattice interval of the lattice pattern 56 is constant. Reference numeral 56A indicates a plurality of intersection points between the first line and the second line. The processor 41 detects the marker M by searching for a morphological feature of the marker M, such as the lattice pattern 56, from the operative field image 21. Of course, the marker M may be detected by using an artificial intelligence (AI) technique using a machine learning model instead of a rule-based method such as the pattern matching.

The processor 41 detects the marker M to specify a region in which the marker M exists in the operative field image 21. In step S1300, first, the position and the posture of the distal end part 14B are estimated based on the morphological feature such as the lattice pattern 56 of the marker M, and at last, the position/posture information indicating the position and the posture of the guide groove 29 is derived. A specific method thereof is as follows.

Figs. 10 and 11 conceptually show a correspondence relationship between the position and the posture of the distal end part 14B of the ultrasound probe 14 having the marker M in the operative field image 21, and the position and the posture of the distal end part 14B in the operative field SF defined as a three-dimensional space. A Zin axis of the operative field SF, which is the three-dimensional space, is a direction parallel to an imaging optical axis of the camera 13B, an Xin-Yin plane (hereinafter, simply referred to as an XY plane) is a plane parallel to the imaging surface of the camera 13B and orthogonal to the imaging optical axis. In Figs. 10 and 11, the XY plane is parallel to the screen of the operative field image 21. The operative field image 21 is a projection image obtained by projecting the operative field SF from one viewpoint. Therefore, as described below, the processor 41 can estimate the position and the posture of the distal end part 14B in the operative field SF based on the marker M detected from the inside of the operative field image 21.

Fig. 10 shows a state in which the axial direction of the distal end part 14B of the ultrasound probe 14 is orthogonal to the imaging optical axis of the camera 13B in the operative field SF that is the three-dimensional space (more specifically, a state in which the axis AX of the distal end part 14B is parallel to the Xin axis). In a case in which the distal end part 14B of the ultrasound probe 14 is in such a posture, in the operative field image 21, the orthogonal lines of the lattice pattern 56 of the marker M are in a state of being parallel to the Xin axis and the Yin axis, and the intervals between the adjacent intersection points 56A are also the same.

On the other hand, Fig. 11 shows a state in which the axial direction of the distal end part 14B of the ultrasound probe 14 is not orthogonal to the imaging optical axis of the camera 13B in the operative field SF that is the three-dimensional space and is inclined in a depth direction parallel to the imaging optical axis. The posture shown in Fig. 11 is a state in which the axis AX of the distal end part 14B is rotated by approximately -15° about the Yin axis from the posture shown in Fig. 10. In a case in which the distal end part 14B is in such a posture, in the operative field image 21, among the lines of the lattice pattern 56 of the marker M, the line extending in the circumferential direction is shorter as the line is farther from the camera 13B in the depth direction. Further, the intervals between the adjacent intersection points 56A are also shorter as the intersection points 56A are further away from the camera 13B.

In this way, the form of the marker M shown in the operative field image 21 is changed in accordance with the posture of the distal end part 14B. The processor 41 estimates the posture of the distal end part 14B of the ultrasound probe 14 in the operative field SF based on the form of the marker M in the operative field image 21. Specifically, the orientation of the axis AX of the distal end part 14B in the operative field SF, that is, the axial direction of the distal end part 14B is detected as the posture of the distal end part 14B.

In addition, in a case in which the position of the distal end part 14B is changed in the operative field SF, the position of the marker M shown in the operative field image 21 is also changed. The processor 41 estimates the position of the distal end part 14B in the operative field SF based on the position of the marker M. The position of the distal end part 14B is, for example, a position of the reference point of the distal end part 14B, such as a distal end position of the distal end part 14B. Further, an imaging distance from the camera 13B to the marker M in the operative field SF (that is, a distance in the Zin axis direction parallel to the imaging optical axis) can be calculated based on a focal length of the camera 13B and the size of the marker M shown in the operative field image 21. The processor 41 can derive position coordinates of the reference point of the distal end part 14B in the operative field SF based on the dimensional information of the distal end part 14B including the imaging distance and the known dimensions of the marker M.

The processor 41 estimates the position and the posture of the guide groove 29 of the ultrasound probe 14 in the operative field SF based on the position and the posture of the distal end part 14B derived in this way and the positional relationship of the guide groove 29 in the distal end part 14B of the ultrasound probe 14 included in the dimensional information of the ultrasound probe 14.

In the position/posture information of the example of Fig. 10, for example, the position of the distal end part 14B of the ultrasound probe 14 is information such as the position coordinates X01, Y01, and Z01 of the reference point of the distal end part 14B in the operative field SF. The posture of the distal end part 14B is defined as the axial direction of the distal end part 14B in the operative field SF, and is, for example, information indicating that the axis AX of the distal end part 14B is parallel to the XY plane and an XZ plane and is orthogonal to a YZ plane.

The position of the first guide groove 29Ais information such as the position coordinates X11, Y11, and Z11 of the reference point in the operative field SF. The position of the second guide groove 29B is information such as the position coordinates X21, Y21, and Z21 of the reference point in the operative field SF. The posture of each of the first guide groove 29A and the second guide groove 29B is defined as the posture with respect to the axis AX, and is the known inclination angle θ1 and inclination angle θ2 as the dimensional information.

In the position/posture information of the example of Fig. 11, for example, the position of the distal end part 14B of the ultrasound probe 14 is information such as the position coordinates X02, Y02, and Z02 of the reference point of the distal end part 14B in the operative field SF. The posture of the distal end part 14B is defined as the axial direction of the distal end part 14B in the operative field SF, and is, for example, information indicating that the axis AX of the distal end part 14B is -15° with respect to the XY plane, is parallel to the XZ plane, and is 75° with respect to the YZ plane.

The position of the first guide groove 29Ais information such as the position coordinates X12, Y12, and Z12 of the reference point in the operative field SF. The position of the second guide groove 29B is information such as the position coordinates X22, Y22, and Z22 of the reference point in the operative field SF. The posture of each of the first guide groove 29A and the second guide groove 29B is defined as the posture with respect to the axis AX, and is the known inclination angle θ1 and inclination angle θ2 as the dimensional information.

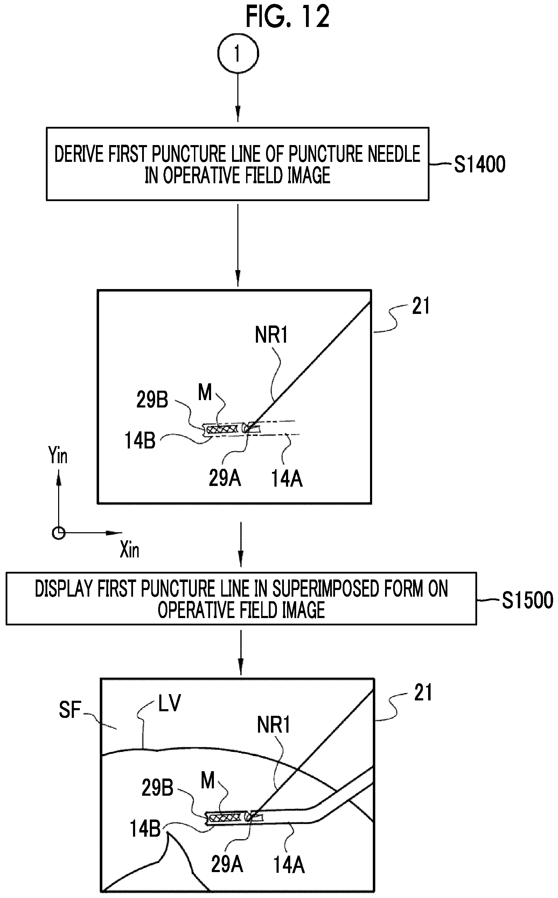

As shown in Fig. 12, in step S1400, the processor 41 derives the first puncture line NR1 of the puncture needle 18 in the operative field image 21 based on the position and the posture of the guide groove 29. The processor 41 derives the first puncture line NR1 that is a straight line along the inclination angle θ1 of the first guide groove 29A in the operative field SF based on the position coordinates of the first guide groove 29A in the operative field SF defined as the three-dimensional space and the inclination angle θ1 of the distal end part 14B with respect to the axis AX. For example, a line segment obtained by using the position of the first guide groove 29A as a base point and adding an angle of the inclination angle θ1 of the first guide groove 29A from the position of the first guide groove 29A is defined as the first puncture line NR1. Then, the processor 41 derives the first puncture line NR1 in the operative field image 21 by converting the first puncture line NR1 into a two-dimensional coordinate system of the operative field image 21. The same applies to the second guide groove 29B.

In addition, whether to display the first puncture line NR1 with respect to the first guide groove 29A or the first puncture line NR1 with respect to the second guide groove 29B can be selected by the medical staff ST via the operation or the setting. The first puncture line NR1 is derived in accordance with the selected guide groove 29. The example shown in Fig. 12 is an example in which the first puncture line NR1 with respect to the first guide groove 29A is displayed.

In step S1500, the processor 41 displays the derived first puncture line NR1 in a superimposed form on the operative field image 21. As described above, the processor 41 executes the display control of displaying the first puncture line NR1 of the puncture needle 18 in a superimposed form on at the position specified in the operative field image 21 based on the position/posture information indicating the position and the posture of the distal end part 14B. As a result, the operative field image 21 in which the first puncture line NR1 is displayed in a superimposed form is displayed on the display 16. The processor 41 repeatedly executes the above-described processing until the display end instruction is input, such as activation end of the medical support device 11.

Since the operative field image 21 is output to the display 16 as the video, the position and the posture of the first puncture line NR1 in the operative field image 21 displayed on the display 16 are also updated by reflecting the positions of the endoscope 13, the ultrasound probe 14, and the like in the real space captured by the camera 13B.

The actions and the effects of the technique of the present disclosure will be described using, as an example, a case in which the liver LV is punctured by the puncture needle 18 with the tumor 27 in the liver LV, which is the organ as the puncture target, as the target position. As shown in Figs. 1 and 5, for example, the medical staff ST adjusts the position and the posture of the ultrasound transducer 14C provided at the distal end part 14B of the ultrasound probe 14 while observing the ultrasound image 22 in which the second puncture line NR2 is displayed in a superimposed form. Specifically, the position and the posture of the ultrasound transducer 14C are adjusted to the position at which the second puncture line NR2 serving as the extension line of the guide groove 29 passes through the tumor 27. The position and the posture of the first puncture line NR1 displayed in a superimposed form on the operative field image 21 are changed in response to the change in the position and the posture of the ultrasound probe 14. In a case in which the second puncture line NR2 is displayed to pass through the tumor 27 in the ultrasound image 22, the second puncture line NR2 indicates an ideal puncture path of the puncture needle 18 in the liver LV. In this state, the position and the posture of the ultrasound probe 14 are fixed during the puncture.

In addition, the first puncture line NR1 in the operative field image 21 in this state indicates, as shown in Fig. 12 as an example, an ideal puncture path of the puncture needle 18 inside the body cavity from the body surface BS of the patient PT to the surface of the liver LV as the puncture target.

While observing the operative field image 21 in which the ideal first puncture line NR1 is displayed in a superimposed form as shown in Fig. 12, the medical staff ST first adjusts the position and the posture of the puncture needle 18 such that the puncture needle 18 is inserted along the ideal first puncture line NR1 from the outside of the body. Specifically, an approximate insertion position NP corresponding to the first puncture line NR1 is searched for, and the body surface BS of the patient PT is punctured by the puncture needle 18 from the insertion position NP. Then, the puncture needle 18 is inserted into the body cavity toward the first guide groove 29A of the ultrasound probe 14 while adjusting the position and the posture of the puncture needle 18 while viewing the display of the first puncture line NR1. Since the first puncture line NR1 is displayed with the first guide groove 29A as a base point, the first guide groove 29A can easily reach by inserting the puncture needle 18 along the first puncture line NR1.

After the puncture needle 18 reaches the first guide groove 29A, the tumor 27 as the target position can be punctured by the puncture needle 18 by puncturing the liver LV with the puncture needle 18 along the second puncture line NR2 while checking the second puncture line NR2 of the ultrasound image 22 as shown in Figs. 1, 2, and 5. As an example, the tumor 27 is cauterized in a state in which the tumor 27 is punctured by the puncture needle 18.

As described above, the medical support device 11 according to the technique of the present disclosure comprises the processor 41, and the processor 41 acquires the operative field image 21 (example of an intracorporeal image) that is captured by the camera 13B (example of an intracorporeal camera) of the endoscope 13 that images the inside of the body of the patient PT (example of a subject), the operative field image 21 including, within the imaging range, the liver LV (example of an organ) punctured by the puncture needle 18, the insertion part 14A of the ultrasound probe 14 (example of a medical device) inserted into the body, and the marker M that is provided at the insertion part 14A and that is image-recognizable. Then, the processor 41 derives the position/posture information including at least one of the position or the posture of the insertion part 14A in the operative field image 21 based on the marker M. Further, the processor 41 executes the display control of displaying the first puncture line NR1 of the puncture needle 18 in a superimposed form at the position specified in the operative field image 21 based on the position/posture information.

In the technique of the present disclosure, a hardware configuration for displaying the first puncture line NR1 in a superimposed form need only be provided with the marker at the insertion part of the medical device, such as the ultrasound probe 14. Therefore, a large-scale device such as a magnetic system is not required. In addition, even in a case in which the medical device in the related art is diverted, the configuration is simpler than a configuration in which a light source for a marker is incorporated because it is necessary to modify only the exterior of the insertion part. Therefore, the medical support device 11 can display the first puncture line NR1 of the puncture needle 18 at an appropriate position in accordance with the position or the posture of the medical device, such as the ultrasound probe 14, with a simple configuration.

In the above-described embodiment, the medical device inserted into the body of the patient PT is the ultrasound probe 14 (an example of a medical probe) that can observe the internal structure of the organ, such as the liver LV. The treatment of puncturing the organ inside the body with the puncture needle 18 is often performed using the medical probe that can observe the internal structure of the organ. Therefore, as in the above-described embodiment, the technique of the present disclosure is particularly effective in a case in which the medical probe is used as the medical device. Further, the ultrasound probe 14 is used in combination with the puncture needle 18 relatively frequently. Therefore, the technique of the present disclosure is more effective in a case in which the ultrasound probe 14 is used as the medical probe. It should be noted that, as the medical probe that can observe the internal structure of the organ, a probe other than the ultrasound probe 14 may be used, such as an optical coherence tomography (OCT) probe.

It should be noted that the medical device need not be the medical probe that can observe the internal structure of the organ. For example, as the medical device, a treatment tool may be used, which does not have an internal structure observation function and has only the guide groove 29 of the puncture needle 18 at the distal end part. For example, in a case in which the tumor exists on the surface of the organ, in a case in which the treatment of puncturing the tumor on the surface with the puncture needle 18 is performed, even with the treatment tool that does not have the internal structure observation function, the puncture needle 18 can be appropriately guided as long as the guide groove 29 is provided. In this case, for example, the medical staff ST performs the registration of the guide groove 29 of the treatment tool at a position of the surface of the organ corresponding to the tumor, and in this state, the medical staff ST punctures the tumor with the puncture needle 18 through the guide groove 29.

In addition, in the above-described embodiment, as the medical device, a medical probe such as the ultrasound probe 14 including the guide groove 29 is used. Then, the processor 41 specifies the position at which the first puncture line NR1 is superimposed on the operative field image 21 based on a relative positional relationship between the marker M and the guide groove 29 (for example, the dimensional information of the ultrasound probe 14). The puncture needle 18 is often used in combination with the medical probe including the guide groove 29 for guiding the puncture needle 18. Therefore, the technique of the present disclosure is particularly effective in a case in which the medical probe including the guide groove 29 is used as the medical device.

Further, as the medical device, a treatment tool, such as a simple rod without including the guide groove 29, may be used. In a case in which the tumor exists on the surface of the organ, it is possible to point to the tumor even with such a treatment tool. Even in a case in which the first puncture line NR1 is simply displayed in a superimposed form on the operative field image 21 in which the treatment tool points to the tumor on the surface of the organ, the first puncture line NR1 serves as a guide for checking a puncture direction of the puncture needle 18 or the like. Therefore, the technique of the present disclosure is effective even for a medical device that is a treatment tool without including the guide groove 29.

### Second embodiment

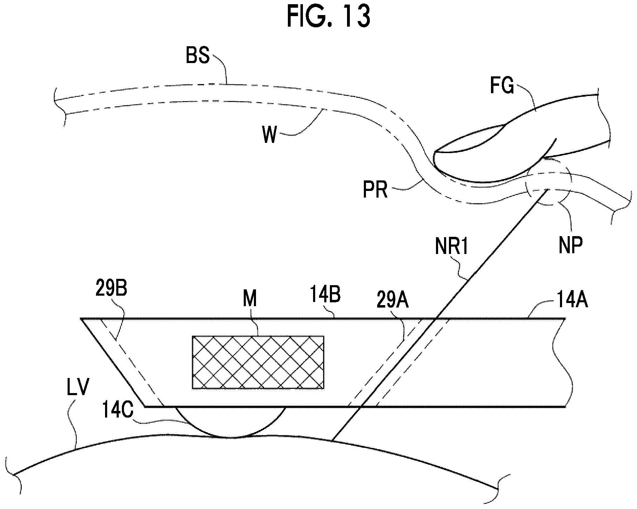

As shown in Fig. 12 of the first embodiment, even in a case in which the first puncture line NR1 is displayed in a superimposed form on the operative field image 21, the information on the depth of the operative field SF is insufficient in the operative field image 21, so that the insertion position NP, which is the intersection point between the first puncture line NR1 and the body surface BS shown in Figs. 5 and 6, may not be able to be accurately understood. The second embodiment shown in Figs. 13 to 19 is an embodiment in which information for understanding the insertion position NP corresponding to the first puncture line NR1 displayed in a superimposed form on the operative field image 21 is provided to a user for the purpose of solving such a problem of the first embodiment.

As an example, as shown in Fig. 13, a case will be considered in which a pressing operation of pressing the body surface BS of the patient PT with a finger FG is performed from the outside of the body. As shown in Figs. 5 and 6, in the endoscopic surgical operation, since the body cavity is inflated with air, the body surface BS bulges in a protruding shape toward the outside of the body from the body cavity. In a case in which such a pressing operation is performed on the body surface BS, a portion corresponding to a pressed position on an interior wall W inside the body cavity of the body surface BS protrudes in a body internal direction, to generate a protrusion portion PR.

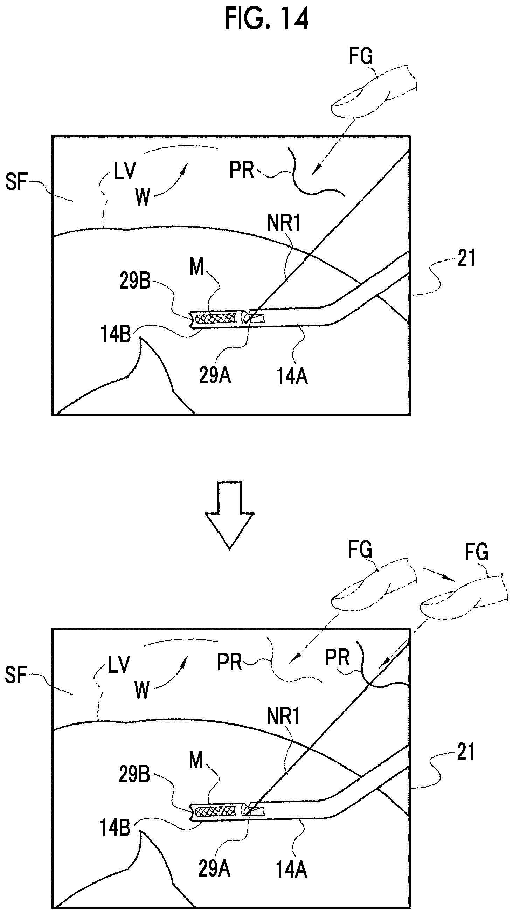

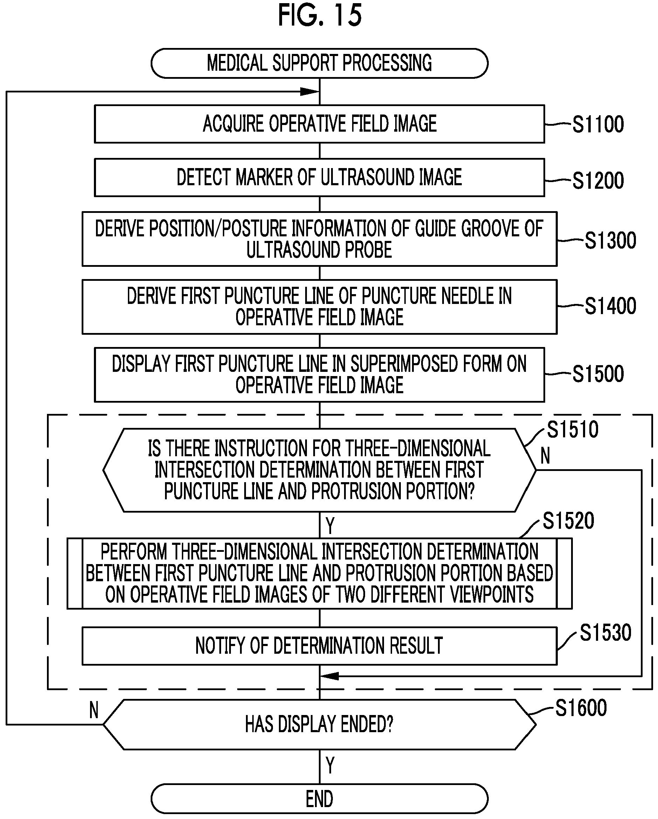

Then, as shown in Fig. 14, for example, while the operative field image 21 in a state in which the first puncture line NR1 is displayed in a superimposed form is displayed on the display 16, the medical staff ST performs the pressing operation on the body surface BS a plurality of times while checking the pressed position of the finger FG on the display 16. In this case, as shown in the operative field image 21 in the lower part of Fig. 14, the medical staff ST can search for the pressed position at which the first puncture line NR1 and the protrusion portion PR intersect with each other. However, as described above, the depth information is insufficient in the operative field image 21. Therefore, even in a case in which the intersection between the first puncture line NR1 and the protrusion portion PR in the operative field image 21 of one viewpoint is simply checked, it is not possible to determine whether or not the first puncture line NR1 and the protrusion portion PR intersect with each other three-dimensionally in the operative field SF that is the three-dimensional space. Therefore, in the second embodiment, a three-dimensional intersection determination of determining whether or not the first puncture line NR1 and the protrusion portion PR intersect with each other in the three-dimensional space is executed. The specific description will be made with reference to Figs. 15 to 19.

Fig. 15 is an example of a flowchart showing an overall processing procedure of the medical support processing in a case in which the three-dimensional intersection determination is performed. The flowchart shown in Fig. 15 is the same as the flowchart in the first embodiment shown in Fig. 8 in that steps S1100 to S1500 and step S1600 are performed. Hereinafter, the difference will be mainly described. The difference is steps S1510 to S1530 shown by broken lines.

As shown in Fig. 15 as an example, the three-dimensional intersection determination is started in a case in which an operation instruction of the medical staff ST is input in step S1500 in a state in which the first puncture line NR1 is displayed in a superimposed form. The medical staff ST inputs an operation instruction to start the three-dimensional intersection determination in a state in which the pressing operation on the body surface BS with the finger FG is performed. In step S1510, the processor 41 waits for the input of the operation instruction, and in a case in which the operation instruction is input (Y in step S1510), the processor 41 proceeds to step S1520 and executes the three-dimensional intersection determination.

The three-dimensional intersection determination in step S1520 is the three-dimensional intersection determination between the first puncture line NR1 and the protrusion portion PR based on the operative field images 21 of two different viewpoints.

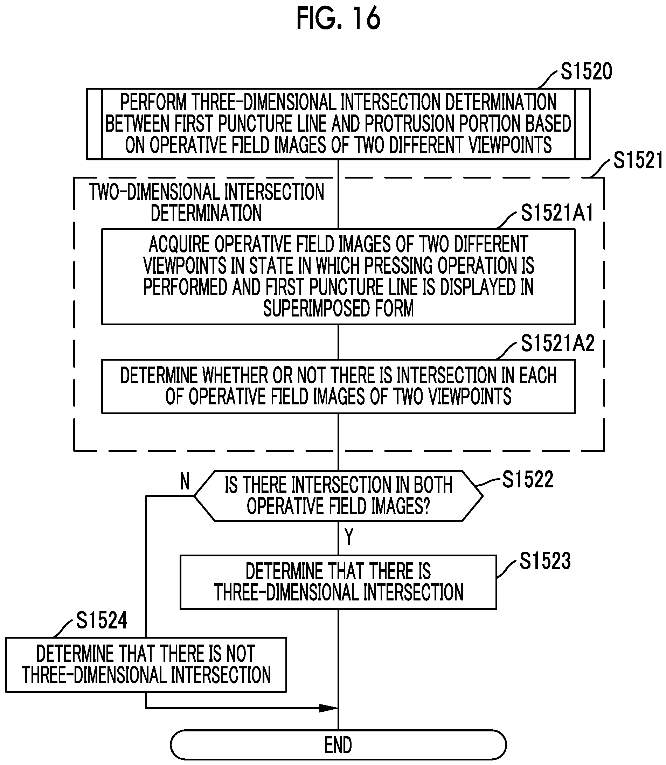

As shown in Fig. 16, first, in step S1521, the processor 41 executes a two-dimensional intersection determination based on the operative field images 21 of the two viewpoints.

First, in step S1521A1, the processor 41 acquires the two operative field images 21 of the first viewpoint VP1 and the second viewpoint VP2, which are different from each other, in a state in which the pressing operation is performed and the first puncture line NR1 is displayed in a superimposed form.

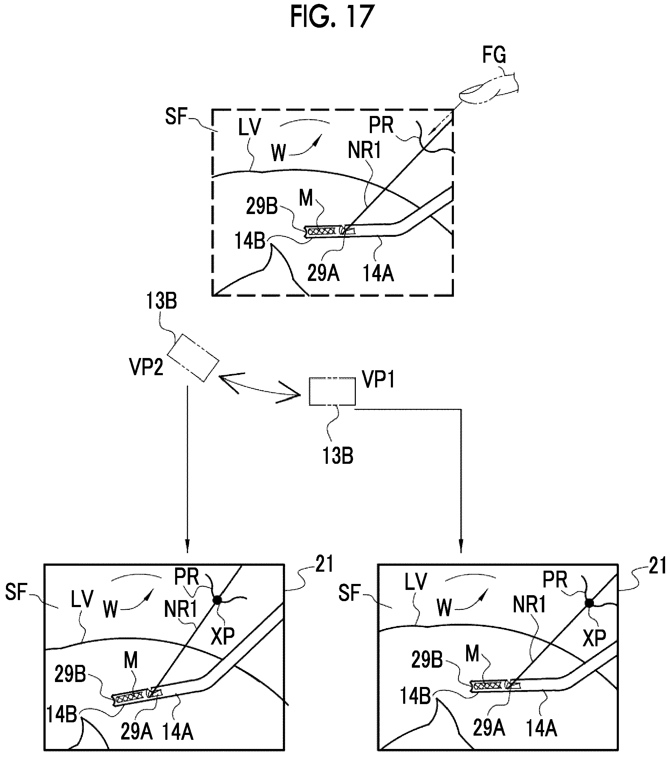

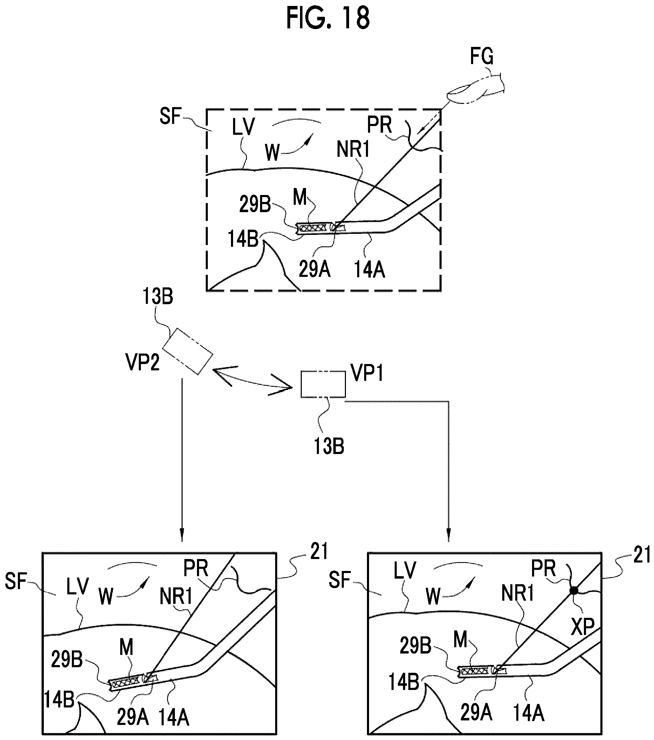

As schematically shown in Figs. 17 and 18, in a state in which the pressing operation on the body surface BS with the finger FG is performed, the medical staff ST changes the viewpoint of the camera 13B by operating the endoscope 13. As an example, before and after the change from the first viewpoint VP1 to the second viewpoint VP2, the medical staff ST inputs, to the processor 41, whether the current viewpoint position is the viewpoint position before the change or the viewpoint position after the change. As a result, the processor 41 can acquire the two operative field images 21 of the first viewpoint VP1 and the second viewpoint VP2 before the change.

It should be noted that, instead of the method of detecting the viewpoint change of the camera 13B via the manual operation, the processor 41 may detect the viewpoint change of the camera 13B due to the movement by the image analysis. In this case, for example, the processor 41 stores a certain number of past frames acquired as the video in advance, and in a case in which the viewpoint change is detected, acquires the front and rear frames as the two operative field images 21 of the first viewpoint VP1 and the second viewpoint VP2, respectively.

Next, the processor 41 proceeds to step S1521A2 to determine whether or not the first viewpoint VP1 and the second viewpoint VP2 intersect with each other in each of the operative field images 21.

In step S1521A2, the processor 41 first detects the protrusion portion PR in each operative field image 21. The detection of the protrusion portion PR is performed by, for example, an image analysis method using the pattern matching or the machine learning model. The interior wall W has undulations even in a state in which the pressing operation with the finger FG is not performed, but as described above, in the endoscopic surgical operation, the body cavity is inflated with air, so that the interior wall W bulges in a protruding shape toward the outside of the body as a whole. In such a state, the pressing operation is performed by the finger FG, so that the protrusion portion PR generated in the pressing operation protrudes in a direction opposite to the bulging direction due to the inflation with air. Therefore, since the protrusion portion PR has a feature different from natural undulations in the size and the shape, the protrusion portion PR can be detected by the image analysis method.

Of course, the protrusion portion PR may be detected by using the visual check work of the medical staff ST, in addition to the method of detecting the protrusion portion PR via the image analysis. For example, the display 16 is configured by a touch panel, and the medical staff ST visually checks the position of the protrusion portion PR in the operative field image 21 displayed on the touch panel. Then, the processor 41 may detect the protrusion portion PR based on the input of an operation of designating the position of the protrusion portion PR from the touch panel via the medical staff ST.

In a case in which the processor 41 detects the protrusion portion PR, the processor 41 detects an intersection point XP between the protrusion portion PR and the first puncture line NR1 in each of the operative field images 21 of the first viewpoint VP1 and the second viewpoint VP2 different from each other. Since the processor 41 stores the position of the first puncture line NR1 in the operative field image 21 as the known information, the processor 41 detects the intersection point XP by collating the position of the detected protrusion portion PR in the operative field image 21 with the position of the first puncture line NR1.

The processor 41 performs such a two-dimensional intersection determination in step S1521, and determines the three-dimensional intersection in and after step S1522 based on the determination result of the two-dimensional intersection determination.

As shown in Fig. 17, in a case in which the intersection point XP is detected in both the operative field images 21 of the first viewpoint VP1 and the second viewpoint VP2, which are different from each other, it is considered that the protrusion portion PR and the first puncture line NR1 intersect with each other in the operative field SF, which is the three-dimensional space inside the body. In this case, the intersection point between the first puncture line NR1 and the interior wall W side is an appropriate insertion position NP.

In step S1522, the processor 41 determines whether or not the protrusion portion PR and the first puncture line NR1 intersect with each other in both the operative field images 21 of the first viewpoint VP1 and the second viewpoint VP2 different from each other. In a case in which an affirmative determination is made in step S1522 (Y in step S1522), the processor 41 proceeds to step S1523 and determines that the protrusion portion PR and the first puncture line NR1 intersect with each other three-dimensionally. That is, the processor 41 determines that the protrusion portion PR and the first puncture line NR1 intersect with each other in the operative field SF that is the three-dimensional space inside the body.

Meanwhile, in the example shown in Fig. 18, the intersection point XP is detected in the operative field image 21 of the first viewpoint VP1, but the intersection point XP is not detected in the operative field image 21 of the second viewpoint VP2. In this way, in a case in which the intersection point XP is not detected in both the first viewpoint VP1 and the second viewpoint VP2, which are different from each other, it is considered that the protrusion portion PR and the first puncture line NR1 do not intersect with each other in the operative field SF that is the three-dimensional space inside the body. In this case, the intersection point between the first puncture line NR1 and the interior wall W side is not the appropriate insertion position NP.

In a case in which a negative determination is made in step S1522 (N in step S1522), the processor 41 proceeds to step S1524 and determines that the protrusion portion PR and the first puncture line NR1 do not intersect with each other three-dimensionally. That is, the processor 41 determines that the protrusion portion PR and the first puncture line NR1 do not intersect with each other in the operative field SF that is the three-dimensional space inside the body.

Here, the intersection point XP is, for example, a point at which the protrusion portion PR and the first puncture line NR1 intersect with each other within a range of a region from a vertex of the protrusion portion PR to an edge thereof. The intersection point XP may be, for example, a point at which the vertex portion of the protrusion portion XP and the first puncture line NR1 intersect with each other. However, in the present example, the intersection with the first puncture line NR1 in the region having the width up to the edge of the protrusion portion PR is also detected as the intersection point XP. In this way, the range in which the intersection point XP is detected has a width, and thus the insertion position NP to be decided also has a width, but there is no problem in practice. This is because, since the body surface BS and the interior wall W have flexibility, it is possible to correct the puncture direction of the puncture needle 18 even in a case in which the insertion position NP of the puncture needle 18 is shifted to some extent.

After the three-dimensional intersection determination in step S1520 ends, the processor 41 gives a notification of the determination result in step S1530 shown in Fig. 15. As the method of giving the notification of the determination result, for example, a method of making the first puncture line NR1 blink, changing the color, thickening the line, increasing the brightness, or the like on the operative field image 21 can be considered. In addition, a message indicating the three-dimensional intersection may be displayed. Instead of the visual method, for example, a message indicating the three-dimensional intersection may be given in a voice, or a warning sound may be emitted.

The medical staff ST understands the pressed position on the body surface BS at which the first puncture line NR1 and the protrusion portion PR intersect with each other three-dimensionally by checking the determination result of step S1530, and as a result, the medical staff ST can understand the appropriate insertion position NP of the puncture needle 18.

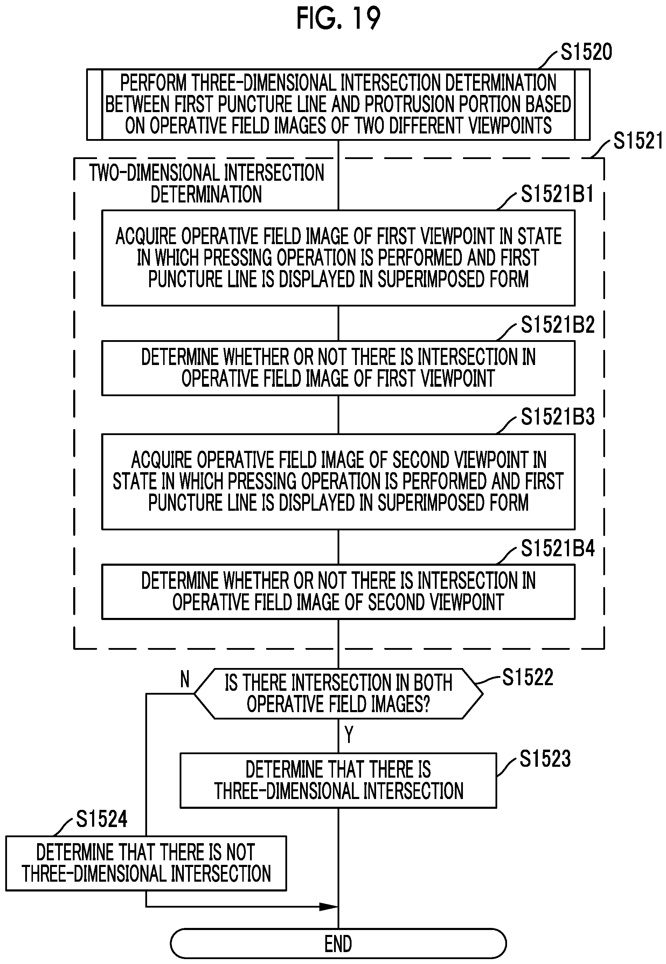

It should be noted that, as the procedure of the two-dimensional intersection determination in step S1521, a procedure shown in Fig. 19 may be used, for example, instead of the procedure shown in Fig. 16. In the procedure shown in Fig. 16, in a state in which the pressing operation with the finger FG is continued, the processor 41 acquires the two operative field images 21 of the first viewpoint VP1 and the second viewpoint VP2, which are different from each other, and then performs two-dimensional intersection determination for each of the acquired two operative field images 21. The operative field image 21 of the first viewpoint VP1 and the operative field image 21 of the second viewpoint VP2 are examples of a first intracorporeal image and a second intracorporeal image acquired in a state in which the operation such as the pressing operation is continued.

On the other hand, in the procedure shown in Fig. 19, the operation includes two operations of a first operation and a second operation performed at different timings on the same position, and the operative field images 21 of different viewpoints are acquired at the respective timings. That is, first, in step S1521B1, the processor 41 performs the first operation, which is the first pressing operation, and acquires a first operative field image 21 (an example of a first intracorporeal image) of the first viewpoint VP1 in a state in which the first puncture line NR1 is displayed in a superimposed form. In step S1521B2, the processor 41 determines the two-dimensional intersection between the protrusion portion PR and the first puncture line NR1 in the first operative field image 21. This determination is an example of a first determination.

Next, in step S1521B3, the processor 41 performs the second operation, which is the second pressing operation, and acquires a second operative field image 21 (an example of a second intracorporeal image) of the second viewpoint VP2 in a state in which the first puncture line NR1 is displayed in a superimposed form. Here, the pressed position at which the second pressing operation is performed is the same position as the pressed position of the first pressing operation. In step S1521B4, the two-dimensional intersection between the protrusion portion PR and the first puncture line NR1 in the second operative field image 21 is determined. This determination is an example of a second determination. The processing in and after step S 1523 is the same as the processing of the procedure of Fig. 16.

As described above, the procedure of the two-dimensional intersection determination in step S 1521 may be the procedure shown in Fig. 16 or the procedure shown in Fig. 19.

As described above, in the second embodiment, the processor 41 acquires the two operative field images 21 that are examples of the intracorporeal image in a state in which the pressing operation, which is an example of the operation of causing the change in the interior wall W inside the body, is performed from the outside of the body and the first puncture line NR1 indicating the puncture path is displayed in a superimposed form, the operative field images 21 being captured from at least the two different viewpoints of the first viewpoint VP1 and the second viewpoint VP2. Then, the processor 41 determines whether or not the protrusion portion PR, which is an example of the change position at which the interior wall W is changed in the three-dimensional space of the inside of the body, and the first puncture line NR1 intersect with each other based on the two operative field images 21, and gives a notification of the determination result. Therefore, in the second embodiment, the information on the first puncture line NR1 in consideration of the depth information, which is insufficient in the first embodiment, can be provided. As a result, the medical staff ST can understand the appropriate insertion position NP at which the first puncture line NR1 and the interior wall W intersect with each other three-dimensionally.

In addition, in the example described above, in a case in which the protrusion portion PR, which is an example of the change position at which the change occurs in the interior wall W, and the first puncture line NR1, which is the puncture path displayed in a superimposed form, intersect with each other two-dimensionally in both of the two operative field images 21, the processor 41 determines that the protrusion portion PR and the first puncture line NR1 intersect with each other in the three-dimensional space. As described above, since the three-dimensional intersection is determined by determining the two-dimensional intersection in the two operative field images 21, the three-dimensional intersection can be determined by simple processing.

In the second embodiment, the pressing operation is an example of an "operation of causing a change in the interior wall W inside the body", and the protrusion portion PR is an example of a "change position" at which the interior wall W is changed. The operation of causing the change in the interior wall W may be an operation other than the pressing operation, and may be, for example, an operation such as light irradiation from the outside of the body or coloring agent injection. The pressing operation is an operation of causing a shape change in the interior wall W. The light irradiation is, for example, an operation of irradiating the body surface BS with light using a laser pointer or the like to cause a brightness change in the interior wall W portion corresponding to an irradiation position. In this case, the position at which the brightness change occurs is a "change position". The coloring agent injection is an operation of causing a color change in the interior wall W by injecting the coloring agent into the body surface BS. In this case, the position at which the color change occurs is a "change position". For example, the processor 41 can detect a change position at which the brightness change or the color change occurs based on a pixel value of the operative field image 21 (including brightness information and color information). As described above, the operation of causing the change in the interior wall W inside the body need only be an operation of causing some change in the interior wall W, which is performed from the outside of the body.

It should be noted that the pressing operation is used as the operation of causing the change in the interior wall W, and thus the change in the interior wall W can be caused by a simple operation without using the tool. In addition, since the protrusion portion PR is the shape change, the image recognition may be easily performed as compared with the brightness change or the color change.

Although the case has been described in which the three-dimensional intersection determination is performed by using the operative field images 21 of the two viewpoints, the operative field images 21 of three or more viewpoints may be used.

### Modification example 1 of second embodiment: operation history

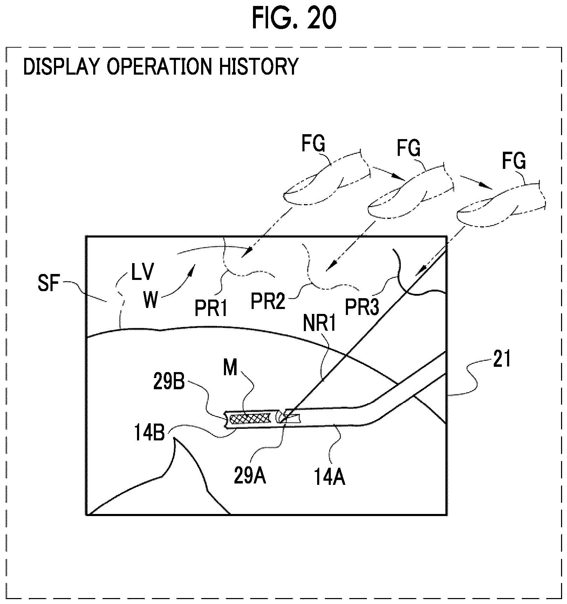

As shown in Fig. 20 as an example, in a case in which the operation of causing the change in the interior wall W is performed a plurality of times, such as the pressing operation with the finger FG, the processor 41 may display an operation history, which is a history of the change position of each of the plurality of times of the operations, in a superimposed form on the operative field image 21. In Fig. 20, an operation history of the pressing operation is shown as an example of the history of the change position.

In a case in which the processor 41 detects the protrusion portion PR, the processor 41 transitorily records the detection position of the detected protrusion portion PR, and displays the protrusion portions PR (shown as PR1, PR2, and PR3 in Fig. 20) generated by the plurality of times of the operations performed in the past, in a superimposed form on each of the detection positions of the operative field image 21. As a result, the medical staff ST can check the relative positional relationship between the past pressed position pressed by himself/herself and the first puncture line NR1. Therefore, repeatedly pressing the same pressed position is suppressed, and thus the search efficiency of the insertion position NP is improved.

### Modification example 2 of second embodiment: following viewpoint change of camera

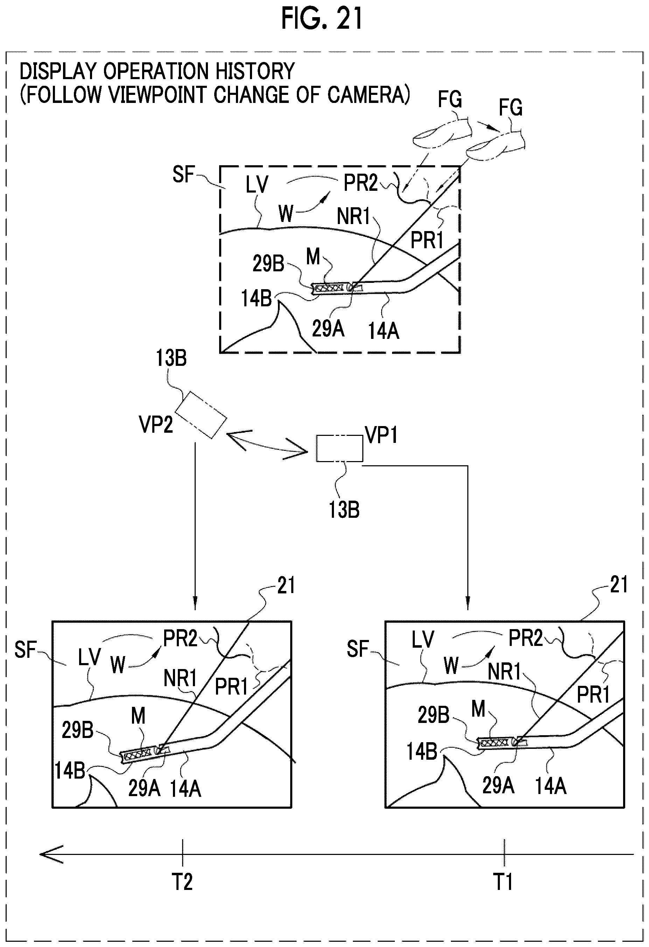

In addition, as shown in Fig. 21 as an example, the operation history shown in Fig. 20 may be made to follow the viewpoint change of the camera 13B. That is, in a case in which the viewpoint of the intracorporeal camera is moved among the plurality of times of the operations such as a plurality of times of the pressing operations, the processor 41 displays a history of the protrusion portion PR, which is an example of the history of the change position, by reflecting the movement amount of the viewpoint.

As an example, as shown in Fig. 21, a case will be considered in which the viewpoint of the camera 13B is changed from the first viewpoint VP1 to the second viewpoint VP2 over time in a case in which the protrusion portion PR1 and the protrusion portion PR2 are displayed as the operation history. In this case, the display positions of the protrusion portion PR1 and the protrusion portion PR2, which are displayed in a superimposed form on the first viewpoint VP1 of the operative field image 21 acquired at a time point T1, and the display positions of the protrusion portion PR1 and the protrusion portion PR2, which are displayed in a superimposed form on the operative field image 21 of the second viewpoint VP2 acquired at a time T2, are naturally changed. The processor 41 detects the movement amount of the viewpoint of the camera 13B to correct the display positions of the protrusion portion PR1 and the protrusion portion PR2, which are the past operation history, in accordance with the movement amount.

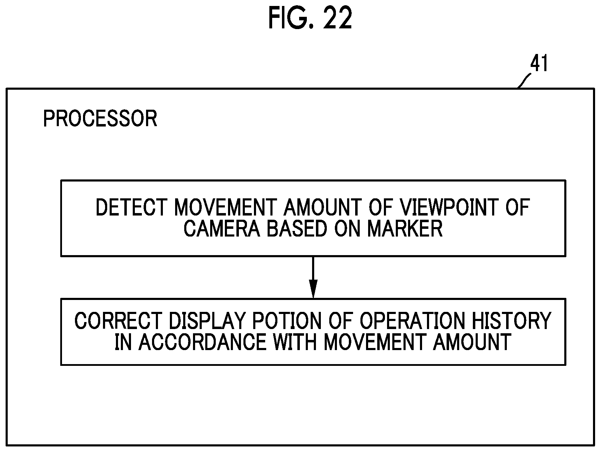

As an example, as shown in Fig. 22, the processor 41 detects the movement amount of the viewpoint of the camera 13B based on the marker M. For example, as shown in Figs. 5 and 6, in a case in which the tumor 27 is punctured by the puncture needle 18, for example, the ultrasound probe 14 is fixed at a position at which the tumor 27 and the second puncture line NR2 overlap in the ultrasound image 22. In a case in which the viewpoint of the camera 13B is moved from the first viewpoint VP1 to the second viewpoint VP2 in a state in which the position of the ultrasound probe 14 is fixed as described above, the appearance of the marker M provided on the distal end part 14B is changed in each of the operative field image 21 of the first viewpoint VP1 and the operative field image 21 of the second viewpoint VP2. The processor 41 derives the position/posture information of the distal end part 14B of the ultrasound probe 14 in the same manner as described with reference to Figs. 10 and 11. Here, since the position of the distal end part 14B is fixed, the change amounts of the position/posture information derived from the operative field image 21 of the first viewpoint VP1 and the position/posture information derived from the operative field image 21 of the second viewpoint VP2 correspond to the movement amount of the viewpoint of the camera 13B. Therefore, the processor 41 can detect the movement amount of the viewpoint of the camera 13B based on the position/posture information of the marker M shown in the two operative field images 21 of the first viewpoint VP1 and the second viewpoint VP2 different from each other. Then, the processor 41 corrects the display position of the operation history in accordance with the detected movement amount. As shown as the display position of the protrusion portion PR, the operation history is an example of a history of the change position of the interior wall W.

In this way, since the history of the change position of the interior wall W is displayed by reflecting the movement amount of the viewpoint of the camera 13B, even in a case in which the viewpoint of the camera 13B is changed, the history of the change position of the interior wall W can be displayed at an appropriate position.

### Modification example 3 of second embodiment

In the second embodiment, in the above-described example, the case has been described in which the accurate information on the insertion position NP corresponding to the intersection point XP between the change position of the interior wall W, such as the protrusion portion PR, and the first puncture line NR1 is presented by the image analysis, but the accurate information can be presented without using the image analysis in some cases.

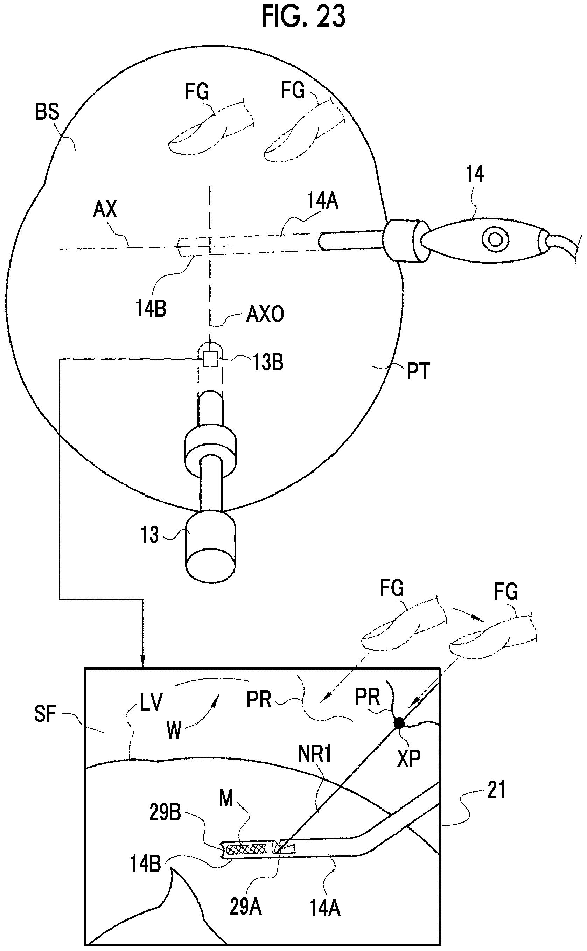

As an example, as shown in Fig. 23, a case will be considered in which the axis AX of the distal end part 14B of the ultrasound probe 14 and an imaging optical axis AXO of the camera 13B of the endoscope 13 are substantially orthogonal to each other. In this case, even in a case in which the pressed position is changed along the direction of the axis AX, the position in the depth direction is not changed. Therefore, in the case in which the intersection point XP at which the protrusion portion PR and the first puncture line NR1 intersect with each other two-dimensionally can be searched for in the operative field image 21 by changing the pressed position along the direction of the axis AX, it can be determined that the intersection point XP is also a point of the three-dimensional intersection. As described above, in a limited case in which the axis AX of the distal end part 14B of the ultrasound probe 14 and the imaging optical axis AXO of the camera 13B of the endoscope 13 are substantially orthogonal to each other, it is possible to determine the three-dimensional intersection without the image analysis.

### Third embodiment

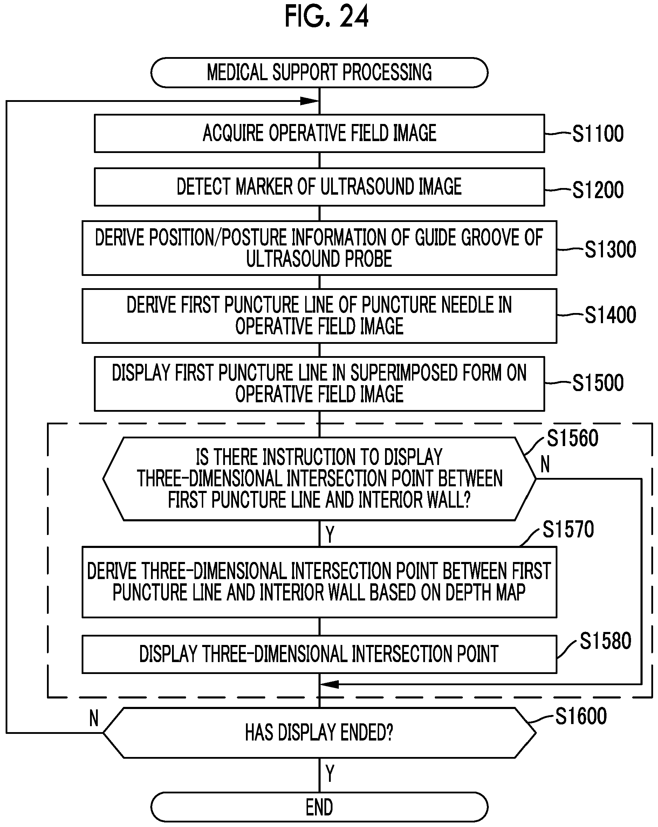

In the third embodiment shown in Figs. 24 to 26 as an example, the processor 41 derives an intersection point between the interior wall W inside the body and the first puncture line NR1 in the three-dimensional space inside the body based on a depth map DMP indicating a distribution of a depth, which is a distance in the depth direction from the viewpoint of the camera 13B. The depth map DMP is an example of "depth information" according to the technique of the present disclosure.

Fig. 24 is a flowchart showing an example of an overall procedure of a medical support processing according to the third embodiment. The flowchart shown in Fig. 24 is different from the flowchart shown in Fig. 15 of the second embodiment in that steps S1560 to S1580 are added instead of steps S1510 to S1530. That is, in Fig. 24 showing an example of the third embodiment, instead of performing the three-dimensional intersection determination between the first puncture line NR1 and the change position (for example, the protrusion portion PR) of the interior wall W, which is performed in the second embodiment, the three-dimensional intersection point XP between the first puncture line NR1 and the interior wall W is displayed based on the depth map DMP. Hereinafter, the third embodiment will be described with reference to the difference from the second embodiment.

In step S1560, the processor 41 receives an instruction to display the three-dimensional intersection point between the first puncture line NR1 and the interior wall W. In a case in which the operation instruction for the intersection point display is input (step S1560: YES), the processor 41 derives the three-dimensional intersection point XP between the first puncture line NR1 and the interior wall W based on the depth map DMP. In step S1560, the three-dimensional intersection point XP is displayed in a superimposed form on the operative field image 21.

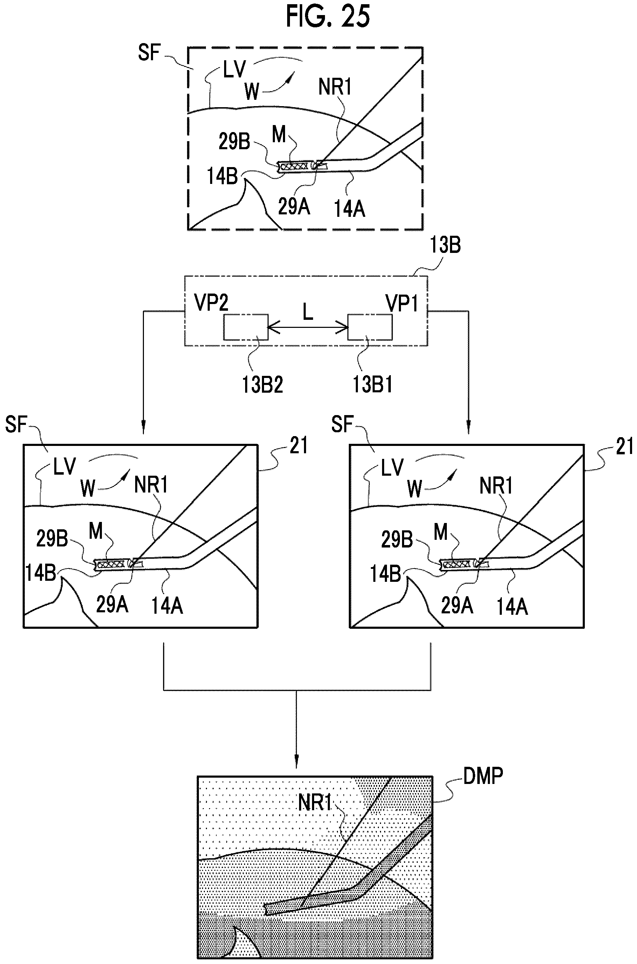

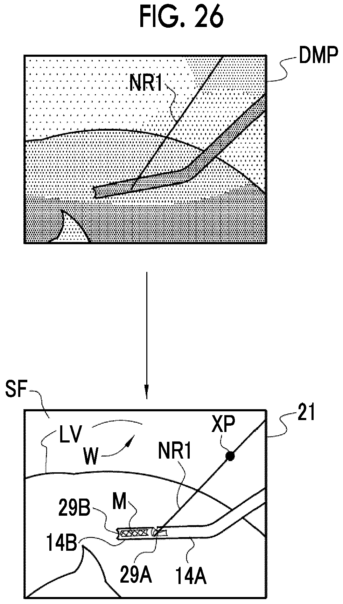

Fig. 25 shows an example of the method of acquiring the depth map DMP. In the example shown in Fig. 25, a stereo camera is used as the camera 13B. The camera 13B as the stereo camera shown in Fig. 25 includes a plurality of cameras 13B1 and 13B2. The camera 13B1 and the camera 13B2 are disposed at an interval, and the interval is a base line length L. The focal lengths of the camera 13B1 and the camera 13B2 are the same as each other. The processor 41 acquires the operative field image 21 captured from the first viewpoint VP1 of the camera 13B1 and the operative field image 21 captured from the second viewpoint VP2 of the camera 13B2. Then, the depth map DMP is generated based on each of the first viewpoint VP1 and the second viewpoint VP2, which are different from each other, of the operative field image 21. The processor 41 generates the depth map DMP based on the principle of the triangulation, the focal length of the camera 13B, the base line length L, and the parallax of the same subject imaged in both the operative field images 21 of the two different viewpoints. The depth map DMP shows the distance in the depth direction of each position in the operative field SF. The coordinates in the operative field SF of the first puncture line NR1 can be acquired by the method using the marker M, which is described with reference to Fig. 10, Fig. 11, and the like. Therefore, as shown in Fig. 26, the processor 41 can derive the intersection point XP between the interior wall W and the first puncture line NR1 based on the depth map DMP.

As described above, in the third embodiment, the processor 41 derives the intersection point XP between the interior wall W and the first puncture line NR1, which is an example of the puncture path, in the three-dimensional space based on the depth map DMP, which is an example of the depth information. Therefore, the three-dimensional intersection point XP between the first puncture line NR1 and the interior wall W can be derived without performing the operation of causing the change in the interior wall W, such as the pressing operation, as in the second embodiment.

The stereo camera of the example shown in Fig. 25 is an example of a "camera configured to estimate the depth" for the operative field SF. As the camera configured to estimate the depth, a camera having a distance sensor as the camera 13B may be used in addition to the stereo camera. The distance sensor is, for example, a sensor using infrared rays. The distance sensor using the infrared rays irradiates the subject with the infrared rays and receives the infrared rays reflected by the subject. The intensity of the infrared rays reflected by the subject is changed in accordance with the distance to the subject. The distance sensor measures a distance by detecting a voltage corresponding to the intensity. The processor 41 can generate the depth map DMP based on the information obtained by the overall scanning of the operative field SF via such a distance sensor.

### Modification example 1 of method of acquiring depth map

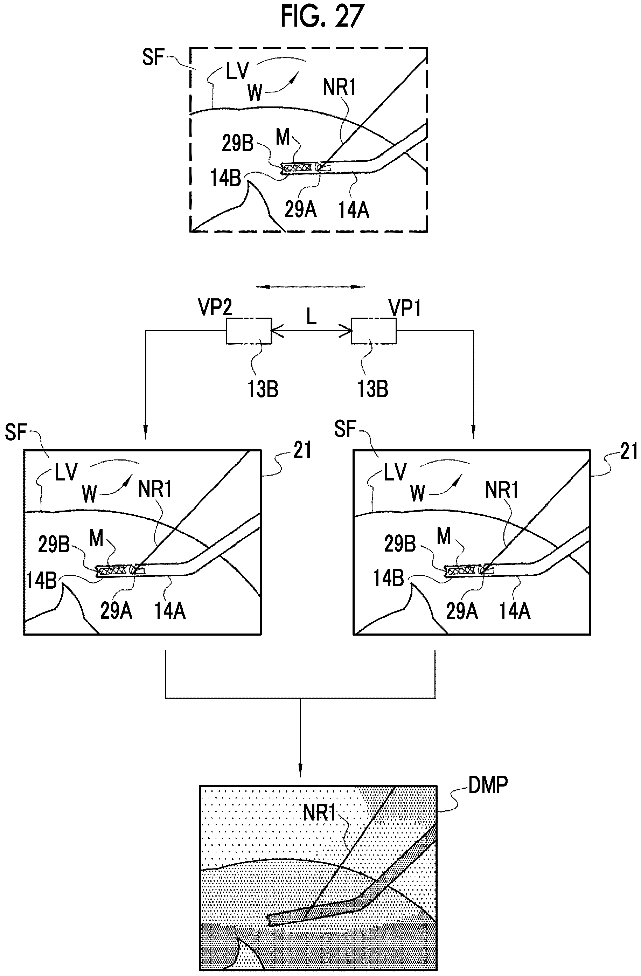

The depth map DMP can be acquired by a method other than the method of estimating the depth using the camera, such as the stereo camera. For example, as shown in Fig. 27, the processor 41 may generate the depth map DMP based on the operative field images 21 of the first viewpoint VP1 and the second viewpoint VP2, the operative field images 21 being acquired by the single camera 13B. That is, the method shown in Fig. 27 is a method of acquiring the operative field images 21 of two different viewpoints by moving one camera 13B instead of using the stereo camera. In this case, the viewpoint is changed by moving the camera 13B. The base line length L corresponds to the movement amount of the camera 13B. As described with reference to Fig. 21, the movement amount of the camera 13B can be detected based on the marker M in a state in which the distal end part 14B is fixed to one position.

It should be noted that, in order to detect the parallax of the subject that is imaged in both the two operative field images 21 of the different viewpoints and the movement amount of the viewpoint of the camera 13B, another marker may be used in addition to or instead of the marker M of the ultrasound probe 14. The other marker is fixed at one position of the operative field SF, and two operative field images 21 of different viewpoints are acquired in this state. Since there is a possibility that the ultrasound probe 14 may move, it is considered that, by using the marker with less possibility of movement than the ultrasound probe 14, it is possible to accurately detect the movement amount of the viewpoint and the parallax, and the accuracy of the depth to be finally acquired is also improved.

The method of generating the depth map DMP from the operative field images 21 of the two viewpoints is the same as in a case of the stereo camera shown in Fig. 25. According to the present example, the depth map DMP can be generated without using the stereo camera.

### Modification example 2 of method of acquiring depth map

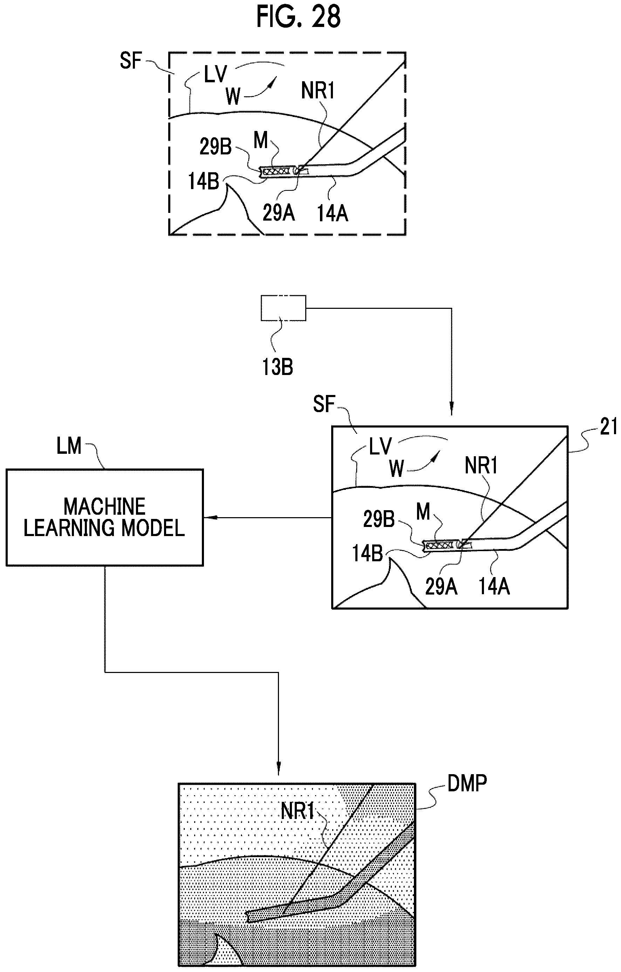

The method of acquiring the depth map DMP is not limited to the method of acquiring the depth map DMP by generating the depth map DMP based on the operative field images 21 of the two viewpoints according to the principle of the triangulation, and as shown in Fig. 28 as an example, the depth map DMP can also be acquired by generating the depth map DMP from one operative field image 21.

In the example shown in Fig. 28, the processor 41 generates the depth map DMP by using a machine learning model LM. The machine learning model LM outputs the depth map DMP by using, for example, the operative field image 21 as input data. The machine learning model LM is trained by using training data composed of a combination of the operative field image 21 and the depth map DMP that is correct answer data. As the machine learning model LM, for example, a convolutional neural network is used. The convolutional neural network includes an encoder that extracts a feature value of an image, and can output the output data in a form of the image in accordance with the feature value extracted by the encoder. As the depth map DMP used as the correct answer data of the training data, for example, the depth map DMP acquired by using the stereo camera is used.

### Modification example of third embodiment

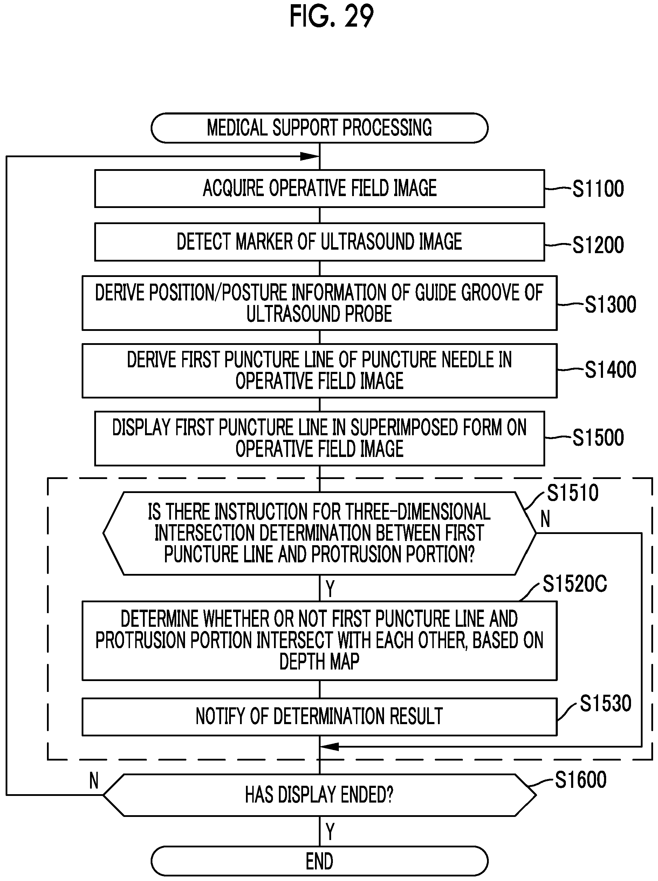

The three-dimensional intersection determination between the first puncture line NR1 and the protrusion portion PR described with reference to Fig. 15 in the second embodiment may be performed by using the depth map DMP. The flowchart shown in Fig. 29 is the same as the flowchart shown in Fig. 15, except that step S1520C is added instead of step S1520 shown in Fig. 15. Hereinafter, the difference will be mainly described.

As described above, the protrusion portion PR is generated on the interior wall W by the pressing operation. The pressing operation is an example of an operation of causing the change in the interior wall W, and the protrusion portion PR is an example of the change position of the interior wall W. The shape change occurs in the interior wall W due to the pressing operation. The processor 41 can understand the information on the depth of the operative field SF by the depth map DMP. As described with reference to Figs. 10 and 11, the three-dimensional coordinates of the first puncture line NR1 in the operative field SF can be derived based on the marker M. In step S1520C, the processor 41 performs the three-dimensional intersection determination between the first puncture line NR1 and the protrusion portion PR based on the depth map DMP.

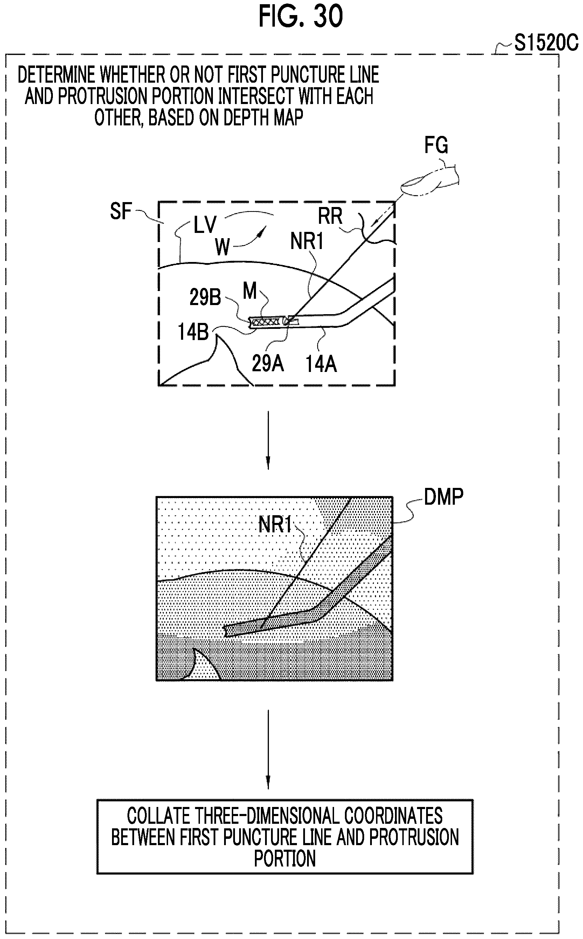

As shown in Fig. 30, in step S1520C, the processor 41 first acquires the depth map DMP by the method shown in Fig. 26 and the like. Then, the processor 41 performs the three-dimensional intersection determination between the first puncture line NR1 and the protrusion portion PR by collating the three-dimensional coordinates of the protrusion portion PR in the first puncture line NR1 and the depth map DMP. As described above, by using the depth map DMP, the three-dimensional intersection determination between the first puncture line NR1 and the shape change position of the interior wall W can be performed.

It should be noted that, in the example of Fig. 30, as the operation of causing the change in the interior wall W, the pressing operation of causing the shape change in the interior wall W has been described as an example, but the operation of causing the change in the interior wall W need not be the operation of causing the shape change. For example, the operation such as the light irradiation or the coloring agent injection may be performed on the interior wall W to cause the brightness change or the color change, as described in the second embodiment. In this case, for example, the processor 41 detects the change position at which the brightness change or the color change occurs from the operative field image 21. As a result, the processor 41 can derive the two-dimensional coordinates of the change position in the operative field image 21. Then, the processor 41 extracts the depth information corresponding to the two-dimensional coordinates of the change position from the depth map DMP, to derive three-dimensional coordinates of the change position. The processor 41 can perform the three-dimensional intersection determination between the first puncture line NR1 and the change position of the brightness or the color by collating the three-dimensional coordinates of the first puncture line NR1 with the three-dimensional coordinates of the derived change position.

### Modification example of method of displaying first puncture line

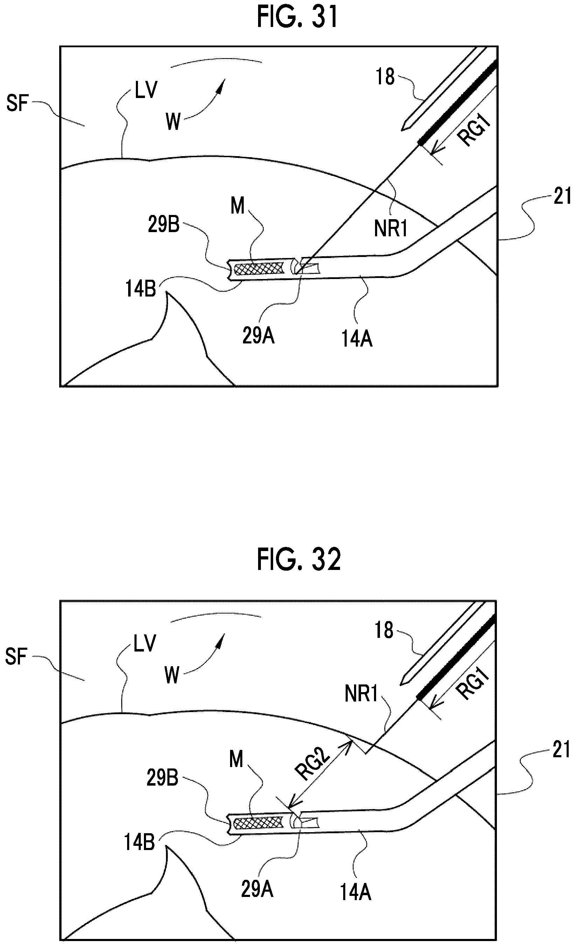

As shown in Figs. 31 and 32, the processor 41 may change the display of a part of the first puncture line NR1. That is, the display mode may be changed in a part of the entire region from one end to the other end of the first puncture line NR1, instead of uniformly changing the display mode.

As shown in Fig. 31 as an example, the processor 41 may highlight a part of the first puncture line NR1 on the interior wall W side. In Fig. 31, a range RG1 is a highlighted range. For the first puncture line NR1, in many cases, the necessity of the display is relatively high immediately after the puncture of the body surface BS. This is because the state in which the needle tip of the puncture needle 18 approaches the guide groove 29 of the ultrasound probe 14 is a state in which the puncture needle 18 can be inserted toward the guide groove 29 of the ultrasound probe 14, and thus the necessity of guiding the insertion of the puncture needle 18 is relatively low in this case. On the other hand, immediately after the body surface BS is punctured by the puncture needle 18, the distance to the guide groove 29 of the ultrasound probe 14 is long. Therefore, it is difficult to understand a relative positional relationship between the puncture needle 18 and the guide groove 29, and there is a relatively high necessity of guiding the insertion of the puncture needle 18 along the first puncture line NR1. By highlighting a part of the first puncture line NR1 on the interior wall W side, the first puncture line NR1 can be made conspicuous in the range RG1 in which the necessity is high.

The range RG1 is, for example, a range of approximately 5 cm from the interior wall W side. In a case in which the inside of the organ is observed with the ultrasound probe 14, the medical staff ST understands an approximate distance from the interior wall W to the organ in the body cavity into which the ultrasound probe 14 is inserted. The approximate distance is, for example, approximately 10 cm. In this case, in a case in which the distance in the range RG1 is approximately 5 cm, the medical staff ST can understand that the needle tip of the puncture needle 18 is located at a position that is advanced by approximately 5 cm from the body surface BS toward the organ in a state in which the needle tip of the puncture needle 18 has reached the end part on the organ side in the range RG1.

In addition, as shown in Fig. 32 as an example, the visibility is made lower in a part of the first puncture line NR1 on the organ side than in a part on the interior wall W side. In the example shown in Fig. 32, the first puncture line NR1 is hidden for a range RG2 of the distance set in advance from the position of the guide groove 29, which is one end of the first puncture line NR1 on the organ side. The medical staff ST inserts the needle tip into the guide groove 29 while checking both the needle tip and the guide groove 29 in a state in which the needle tip of the puncture needle 18 approaches the guide groove 29. In this case, in a case of understanding the positional relationship between the needle tip and the guide groove 29, the first puncture line NR1 may be an obstacle. Therefore, in some cases, the positional relationship between the needle tip of the puncture needle 18 and the guide groove 29 is easily understood by displaying the first puncture line NR1 in a part on the organ side in the range RG2 as shown in Fig. 32.

It should be noted that, in the example shown in Fig. 32, the first puncture line NR1 is hidden in the range RG2, but the first puncture line NR1 need not be hidden. For example, the visibility may be reduced by a method of reducing the brightness as compared with other regions.

In addition, in a case of displaying the first puncture line NR1, the visibility of the range RG2 may not always be reduced, and for example, the visibility may be reduced in a case in which the needle tip of the puncture needle 18 approaches the organ after the body surface BS is punctured by the puncture needle 18. The timing at which the visibility is reduced may be designated manually, or the processor 41 may detect that the puncture needle 18 approaches the organ or the distal end part 14B of the ultrasound probe 14 by performing the image analysis on the operative field image 21.

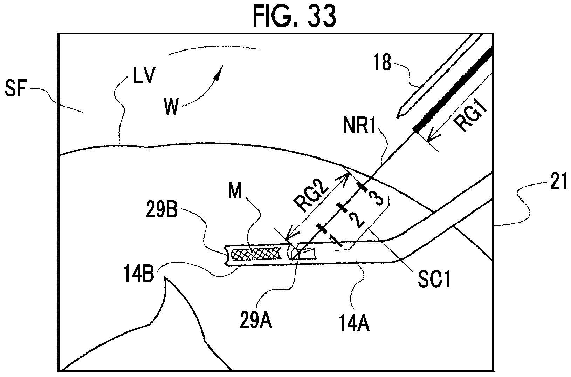

In addition, as shown in Fig. 33, a scale SC1 may be displayed in a part of the first puncture line NR1 on the organ side in the range RG2. The range RG2 in which the scale SC1 is displayed is, for example, a range of approximately 3 cm from the guide groove 29, and the scale SC1 is displayed in the range RG2 in increments of 1 cm. By displaying the scale SC1, it is possible to clearly indicate the degree of the interval between the needle tip of the puncture needle 18 and the guide groove 29 in a case in which the puncture needle 18 approaches the guide groove 29. Since the processor 41 can understand the three-dimensional coordinates of the guide groove 29 in the operative field SF by using the method described with reference to Figs. 10 and 11, the processor 41 can derive the scale SC1 based on the three-dimensional coordinates of the guide groove 29.

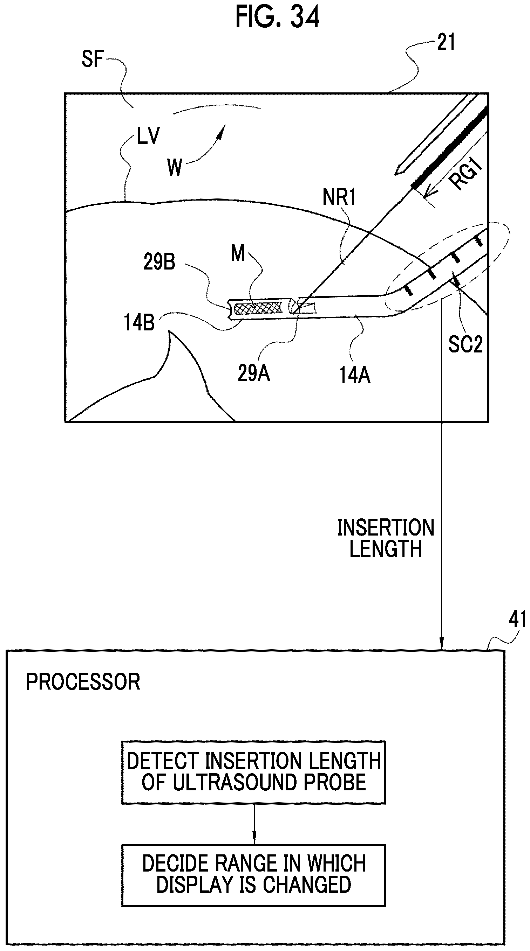

As shown in Fig. 34, the processor 41 may detect an insertion length of the insertion part 14A of the ultrasound probe 14, which is an example of a medical device, and may decide a range in which the display is changed, such as the range RG1, based on the detected insertion length. The insertion part 14A is provided with, for example, a scale SC2 for providing a guide for the insertion length of the insertion part 14A. The insertion part 14A is provided with a bendable part at the distal end part 14B. The scale SC2 is provided in the insertion part 14A at a preset interval (for example, 1 cm interval) from the bendable part toward the base end side. The processor 41 image-recognizes, for example, the scale SC2 from the operative field image 21, and detects the insertion length. In the medical probe such as the ultrasound probe 14, the distal end part 14B is disposed on a surface of the organ. Therefore, the insertion length of the insertion part 14A is an effective clue for understanding the distance from the interior wall W to the surface of the organ. As a result, the processor 41 can appropriately derive the range for displaying the range RG1 or can set the range in which the display of the range RG1 or the like is changed to an appropriate range in accordance with the insertion length.

In addition, the processor 41 may decide a range in which the display of the first puncture line NR1 is changed, based on the designation of the user, such as the medical staff ST. As a result, the range in which the display is changed in the first puncture line NR1 can be set to an appropriate range in response to the user's request.

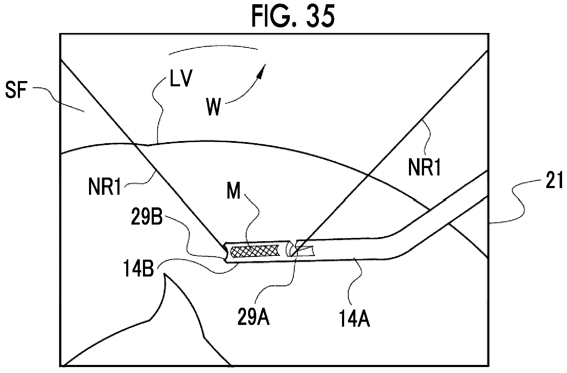

In addition, as shown in Fig. 35, it is preferable that the processor 41 can display a plurality of the first puncture lines NR1 different from each other. As a result, it is possible to display various first puncture lines NR1 depending on the purpose. In the example shown in Fig. 35, the plurality of first puncture lines NR1 corresponding to each of the two types of the first guide groove 29A and the second guide groove 29B provided in the ultrasound probe 14 are displayed.

It should be noted that the processor 41 may display the plurality of first puncture lines NR1 as candidates, allow the medical staff ST, who is the user, to select one thereof, and hide the remaining candidates. Alternatively, in a case in which the plurality of first puncture lines NR1 are displayed and the actual puncture needle 18 is inserted, only the first puncture line NR1 close to the position of the puncture needle 18 may be displayed, and the remaining first puncture lines NR1 may be hidden. The processor 41 can derive the distance between the puncture needle 18 and each of the plurality of first puncture lines NR1 by performing the image analysis on the operative field image 21.

### Fourth embodiment

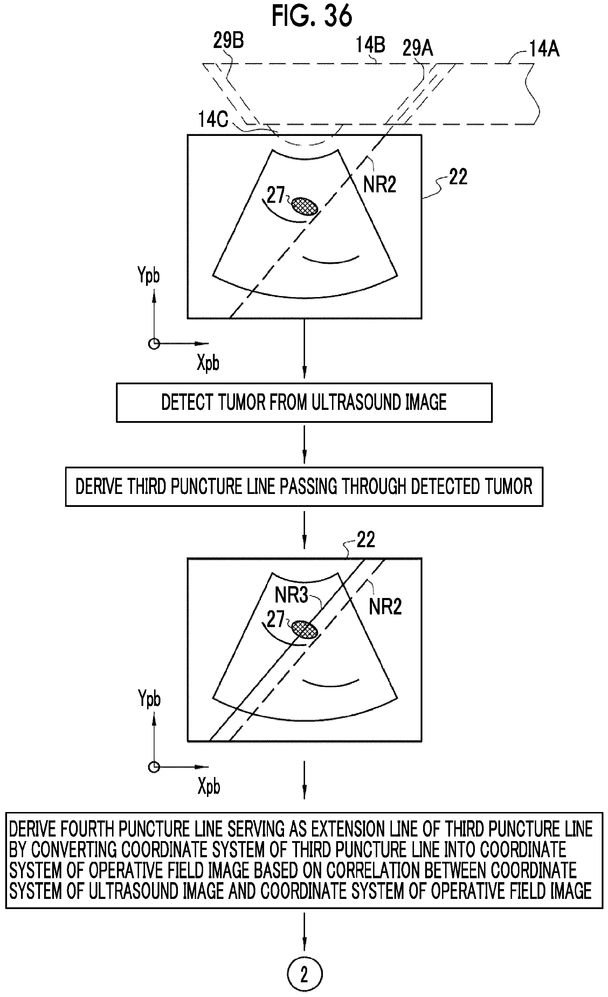

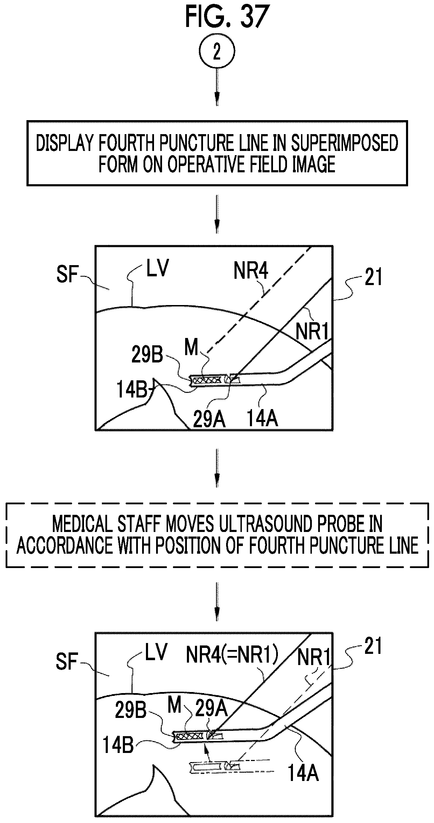

In the fourth embodiment shown in Figs. 36 and 37 as an example, the processor 41 specifies a position in the operative field image 21 at which a fourth puncture line NR4 is superimposed, based on the tumor 27 specified in the ultrasound image 22, and a correlation between the coordinate system of the ultrasound image 22 and the coordinate system of the operative field image 21. The tumor 27 is an example of a target position specified in the internal image of the organ acquired by the medical probe. The correlation is an example of a correlation between a coordinate system of the internal image and a coordinate system of the intracorporeal image, the correlation being derived based on the marker M. The fourth puncture line NR4 is an example of a "puncture path" according to the technique of the present disclosure.

As described with reference to Figs. 10 and 11, it is possible to estimate the position and the posture of the distal end part 14B of the ultrasound probe 14 by using the marker M. The positional relationship of the ultrasound transducer 14C in the distal end part 14B is known from the dimensional information. Therefore, in a case in which the position/posture information of the distal end part 14B can be estimated, it is also possible to estimate the position and the posture of the ultrasound transducer 14C. In this case, the processor 41 can derive the correlation between the coordinate system of the operative field image 21 in which the distal end part 14B of the ultrasound probe 14 is shown, and the coordinate system of the ultrasound image 22 acquired by the ultrasound probe 14.

In the fourth embodiment, first, as shown in Fig. 35, a case will be considered in which the tumor 27 serving as the target position is included in the ultrasound image 22 acquired by the ultrasound probe 14. The second puncture line NR2 is a line set with the guide groove 29 of the ultrasound probe 14 as a base point. In the fourth embodiment, the processor 41 derives a third puncture line NR3 passing through the tumor 27 detected from the ultrasound image 22, separately from the second puncture line NR2. The third puncture line NR3 is derived with reference to the tumor 27 independently of the guide groove 29.

The processor 41 converts the coordinate system of the third puncture line NR3 into the coordinate system of the operative field image 21 based on the correlation between the coordinate system of the ultrasound image 22 and the coordinate system of the operative field image 21, and derives the fourth puncture line NR4 serving as an extension line of the third puncture line NR3. Then, as shown in Fig. 37, the processor 41 displays the derived fourth puncture line NR4 in a superimposed form on the operative field image 21. The fourth puncture line NR4 is an example of a "puncture path" according to the technique of the present disclosure.

The medical staff ST moves the ultrasound probe 14 while looking at the operative field image 21 in accordance with the position of the fourth puncture line NR4, that is, such that the first puncture line NR1 extending from the guide groove 29 of the ultrasound probe 14 as a base point and the fourth puncture line NR4 match each other. Since the fourth puncture line NR4 is a line passing through the tumor 27 that is the target position punctured by the puncture needle 18, in a case in which the first puncture line NR1 matches the fourth puncture line NR4, the guide groove 29 of the ultrasound probe 14 can match an appropriate position. In this state, the medical staff ST can puncture the tumor 27 that is the target position by inserting the puncture needle 18 along the fourth puncture line NR4 (which is also the first puncture line NR1).

According to the fourth embodiment, the internal image acquired by the medical probe, such as the ultrasound probe 14, can be effectively utilized for the registration of the ultrasound probe 14 with respect to the target position of the puncture target of the puncture needle 18.

### Fifth embodiment

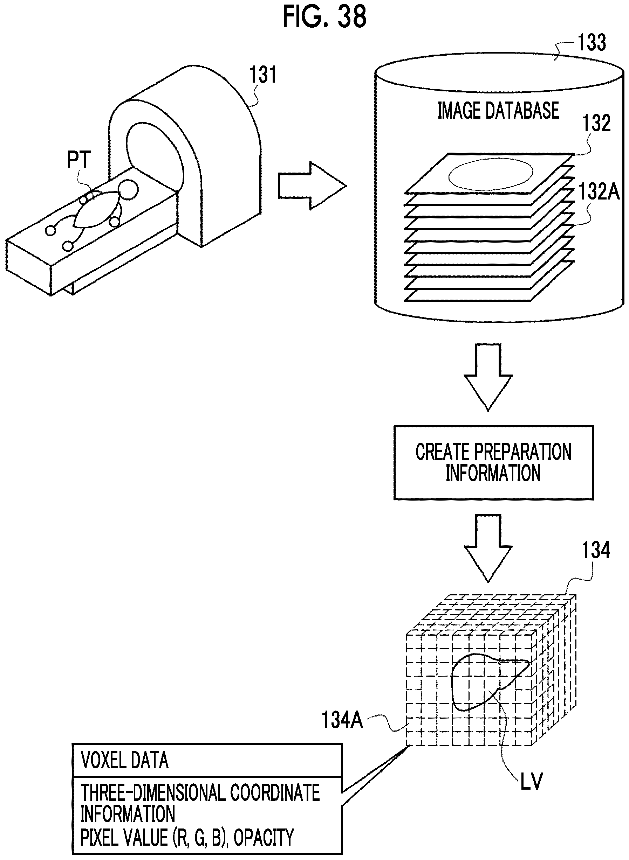

As shown in the fifth embodiment shown in Figs. 38 to 41 as an example, a target puncture line NRT prepared in advance before the surgical operation may be displayed in a superimposed form on the operative field image 21. The target puncture line NRT is an example of a "puncture path" according to the technique of the present disclosure.

### Creation of preoperative preparation information

In the fifth embodiment, for example, before the surgical operation, the preoperative simulation is performed by using 3-dimension (3D) data of a virtual endoscope, and in the preoperative simulation, the target puncture line NRT of the puncture needle 18 is prepared. The target puncture line NRT is an ideal puncture path selected, for example, to avoid a part such as a blood vessel that should not be damaged in the organ or to pass through a part through which the puncture needle 18 can easily pass in a case in which the puncture is performed by the puncture needle 18. In addition, the fifth embodiment is an example in which the ultrasound probe 14 that can observe the internal structure of the organ is used as the medical device. Hereinafter, the description will be made with reference to Figs. 38 to 41.

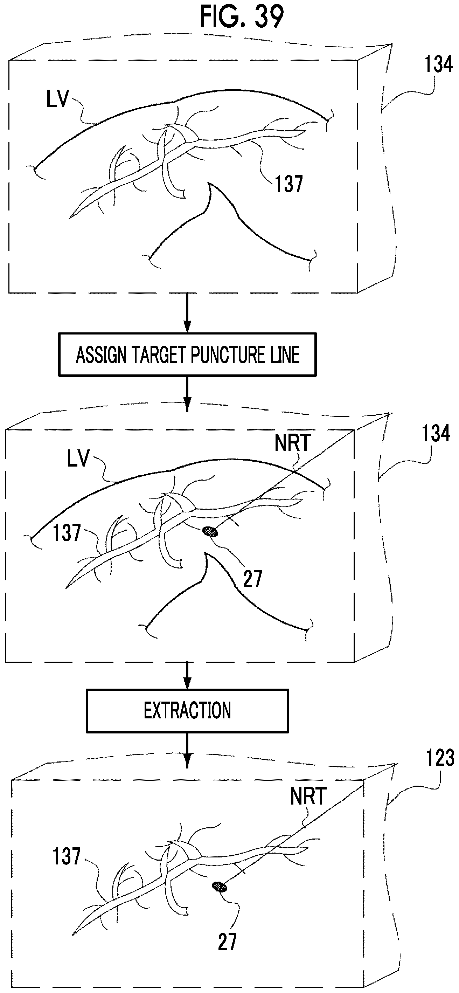

First, a creation method of creating the target puncture line NRT of the puncture needle 18 that punctures the liver LV in the preoperative simulation using the 3D data of the virtual endoscope will be described with reference to Figs. 38 and 39. The 3D data of the virtual endoscope is generated based on a tomographic image group 132 captured in advance by a tomography apparatus 131, such as a computed tomography (CT) apparatus or a magnetic resonance imaging (MRI) apparatus. In a case in which the tomography apparatus 131 is the CT apparatus, a CT value is acquired while rotating a radiation source and a radiation detector about a body axis of the patient PT. The acquisition of the CT value is performed at each position in the body axis direction by performing scanning using the radiation source and the radiation detector in the body axis direction of the patient PT. The CT value is a radiation absorption value inside the body of the patient PT. The tomography apparatus 131 generates a tomographic image 132Aby executing image reconstruction processing based on the CT value acquired in each direction about the body axis. Each tomographic image 132A is a two-dimensional image generated in accordance with a slice thickness in the body axis direction, and the tomographic image group 132 is a set of a plurality of tomographic images 132A corresponding to the respective positions in the body axis direction. The tomographic image group 132 is output to an image database 133, such as a picture archiving and communication system (PACS).

An information processing apparatus (not shown) generates a three-dimensional image 134 that is a set of voxel data 134A by performing 3D modeling that numerically describes a three-dimensional shape of the body of the patient PT based on the tomographic image group 132 obtained by the tomography apparatus 131. The voxel data 134A is a unit of a pixel in the three-dimensional space, and has the three-dimensional coordinate information and the pixel value. The three-dimensional image 134 generated by the 3D modeling is also referred to as three-dimensional volume data or the like. In the tomographic image group 132, the pixel intervals in the respective tomographic images 132A and the slice thicknesses in the respective tomographic images 132A may be different from each other. In this case, for example, in the 3D modeling, the three-dimensional image 134 having the isotropic voxel data 134A in which the lengths in the three dimensions are equal to each other is generated by performing processing of interpolation between the adjacent tomographic images 132A. Here, since the three-dimensional image 134 generated based on the tomographic image group 132 is information created before the surgical operation, the three-dimensional image 134 is referred to as the preoperative 3D image 134 for convenience. The preoperative 3D image 134 is an example of the 3D data of the virtual endoscope, and is further an example of a "three-dimensional image of the organ acquired in advance before a surgical operation" according to the technique of the present disclosure.

The preoperative 3D image 134 is an image capable of reproducing an external shape of the body of the patient PT, an anatomical part such as the organ inside the body, and the internal structure thereof. The preoperative 3D images 134 shown in Figs. 38 and 39 show data of the liver LV as an example of the anatomical part. The preoperative 3D image 134 also includes data capable of reproducing a vascular structure which is an example of the internal structure of the liver LV.

In addition, the preoperative 3D image 134 of the present example is a color image, and the pixel values of red (R), green (G), and blue (B) are given as the pixel values of the voxel data 134A. It should be noted that the preoperative 3D image 134 may be a monochrome image. For example, the pixel value of the voxel data 134A may be represented by only the brightness (Y) based on the CT value. In addition, a value converted from the CT value using a preset look up table (LUT) or an arithmetic expression may be used as the pixel value of the voxel data 134A. Further, the pixel value of the voxel data 134A may be set to a color associated with each specific part such as the organ or the lesion specified in the preoperative 3D image 134. An opacity is also set in the voxel data 134A. The opacity is data used for volume rendering. The rendering is processing of converting a part of the preoperative 3D image 134 into a two-dimensional projection image, and the volume rendering is a rendering method of also projecting internal information of an object included in the preoperative 3D image 134 onto the projection image. By setting the opacity for each voxel data 134A, in a case in which the volume rendering is performed, expressions such as projection in an opaque manner, projection in a translucent manner, and projection in a transparent manner in the projection image can be appropriately used in the internal information.

Information 123 including the target puncture line NRT as shown in Fig. 39 is information prepared in advance before the surgical operation, and is hereinafter referred to as preoperative preparation information. The preoperative preparation information 123 is generated based on the preoperative 3D image 134. The target puncture line NRT is assigned to the preoperative 3D image 134 in the operative simulation performed using the preoperative 3D image 134 before the surgical operation. The preoperative preparation information 123 includes a vascular structure 137 and the tumor 27 as the internal structure of the liver LV as the puncture target, in addition to the target puncture line NRT. The target puncture line NRT and the preoperative preparation information 123 including the internal structure, such as the vascular structure 137 and the tumor 27, are extracted from the preoperative 3D image 134.

As described above, the preoperative preparation information 123 is information in which the three-dimensional position in the preoperative 3D image 134 is defined. The vascular structure 137 included in the preoperative preparation information 123 is used for the registration with the ultrasound image 22 acquired by the ultrasound probe 14.

### Acquisition of ultrasound 3D image

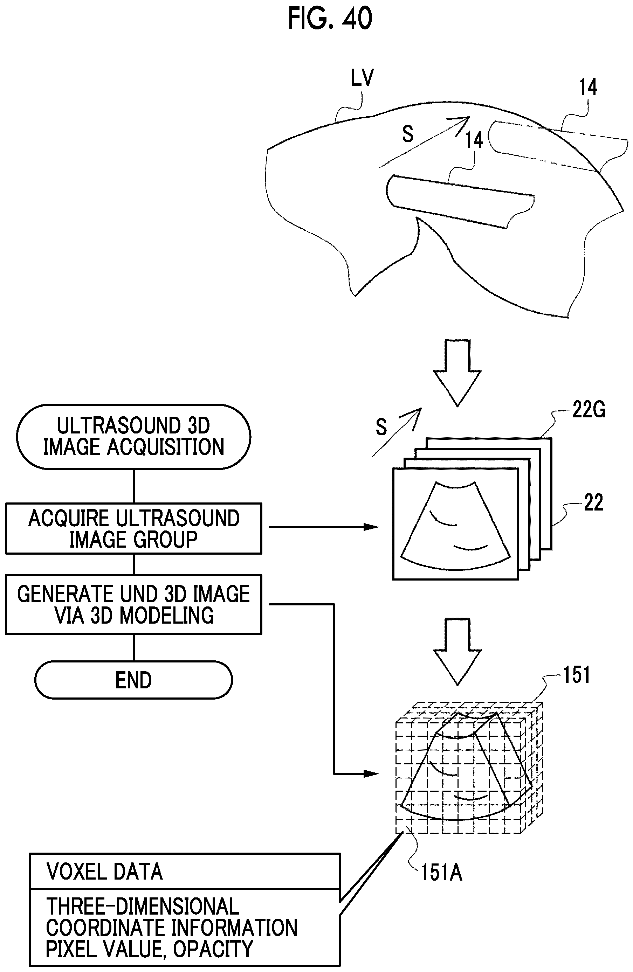

As shown in Fig. 40, the processor 41 acquires an ultrasound 3-dimension (3D) image 151 based on an ultrasound image group 22G which is a set of the ultrasound images 22 captured by the ultrasound probe 14. The processor 41 uses the ultrasound 3D image 151 acquired during the surgical operation, for the registration. The ultrasound 3D image 151 is an example of an "internal image of the organ acquired by the medical probe" according to the technique of the present disclosure.

As shown in Fig. 40, first, in a case in which the ultrasound probe 14 is caused to perform the scanning along the surface of the target part, such as the liver LV, the plurality of ultrasound images 22 at the respective positions on a scanning trajectory S can be acquired. The processor 41 acquires the ultrasound image group 22G and performs the 3D modeling based on the acquired ultrasound image group 22G to generate the ultrasound 3D image 151. The ultrasound image 22 is a tomographic image similarly to the tomographic image 132A shown in Fig. 38, and the ultrasound image group 22G is the same as the tomographic image group 132. In the ultrasound image group 22G, the internal structure of the target part, such as the vascular structure 137 of the liver LV, is also depicted.

The processor 41 generates the ultrasound 3D image 151 by executing the same processing as the 3D modeling described with reference to Fig. 38. The ultrasound 3D image 151 is a set of voxel data 151A, which are pixels in the three-dimensional space, similarly to the preoperative 3D image 134. The voxel data 151Ais also the same as the voxel data 134A shown in Fig. 38, and the three-dimensional coordinates, the pixel value, and the opacity are set.

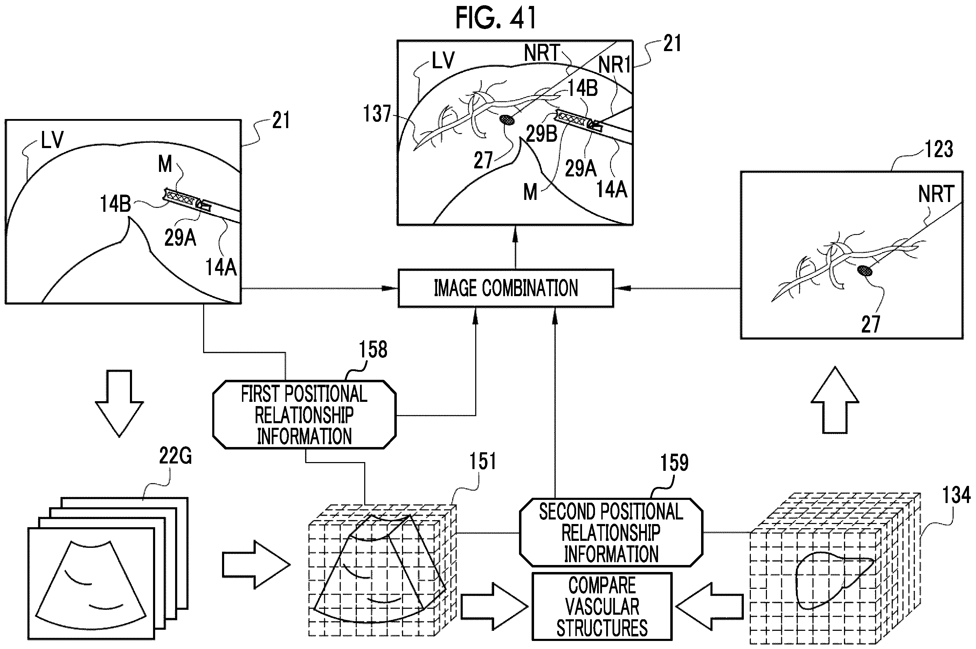

As shown in Fig. 41, the processor 41 derives first positional relationship information 158 that is the correlation between the coordinate system of the operative field image 21 acquired through the endoscope 13 and the coordinate system of the ultrasound 3D image 151, and second positional relationship information 159 that is the correlation between the coordinate system of the ultrasound 3D image 151 and the coordinate system of the preoperative 3D image 134.

First, a method of deriving the first positional relationship information 158 will be described. The processor 41 estimates the position and the posture of the ultrasound transducer 14C based on the marker M of the ultrasound probe 14 shown in the operative field image 21 in the same manner that described in the fourth embodiment. Then, the processor 41 estimates the position and the posture of the ultrasound 3D image 151 in the operative field image 21 based on the estimated position and posture of the ultrasound transducer 14C. The position and the posture of the ultrasound 3D image 151 in the operative field image 21 are the first positional relationship information 158. The first positional relationship information 158 is an example of a "correlation between a coordinate system of the internal image and a coordinate system of the intracorporeal image, the correlation being derived based on the position/posture information" according to the technique of the present disclosure.

Next, a method of deriving the second positional relationship information 159 will be described. The processor 41 compares the vascular structures 137 depicted respectively in the ultrasound 3D image 151 and the preoperative 3D image 134. Specifically, similar structures of both the vascular structures 137 are searched for, and the registration between the ultrasound 3D image 151 and the preoperative 3D image 134 is performed such that both similar structures match each other. The search for the similar structures of the vascular structures 137 may be performed using a rule-based image analysis method such as pattern matching or a method using an AI technique such as semantic segmentation. The processor 41 can derive the second positional relationship information 159 by performing the registration using the vascular structure 137.

Then, the processor 41 combines the preoperative preparation information 123 including the target puncture line NRT generated based on the preoperative 3D image 134 and the operative field image 21, based on the first positional relationship information 158 and the second positional relationship information 159. The target puncture line NRT is superimposed on the tumor 27 at an appropriate position because the registration of the target puncture line NRT is performed by using the vascular structure 137 of the liver LV. The first puncture line NR1 with the guide groove 29 as a base point is displayed on the operative field image 21. As shown in Fig. 37 in the fourth embodiment, the medical staff ST moves the ultrasound probe 14 such that the first puncture line NR1 and the target puncture line NRT match each other. As a result, the position of the ultrasound probe 14 can match an appropriate position for puncturing the tumor 27.

As described above, in a case in which the target puncture line NRT (an example of the puncture path) is set by the preoperative simulation for the preoperative 3D image 134 (an example of the three-dimensional image of the organ) acquired in advance before the surgical operation, the processor 41 superimposes the target puncture line NRT on the operative field image 21 by using the second positional relationship information 159 and the first positional relationship information 158. As a result, it is possible to effectively use the internal image of the medical probe and the result of the preoperative simulation. In the preoperative simulation, for example, it is possible to set an appropriate target puncture line NRT so as not to damage the vascular structure 137. The target puncture line NRT can be provided in the operative field image 21 in this way, so that more appropriate medical support can be provided.

The form of the marker M need not be the lattice form marker as in the present example, and may be a single figure such as a simple polygon or a circle, or a combination of a plurality of these figures. Further, a dot pattern may be used in which the dots are arranged in a matrix at equal intervals.

In addition, in each of the above-described embodiments, cauterization has been described as an example of the function of the puncture needle, but the puncture needle may have a function other than cauterization. Further, although the puncture needle has been described as an example of the treatment tool, in addition to the puncture needle, a treatment tool for injecting a fluorescent agent such as indocyanine green (ICG), a biopsy needle and a forceps used for collecting a tissue for performing a biopsy, or the like may be used.

In each of the above-described embodiments, the body cavity, such as the abdominal cavity or the thoracic cavity, has been described as an example as the inside of the body, but the inside of the body may be an inside of an upper digestive tract such as an esophagus, a lower digestive tract such as an intestine, or a tubular organ such as a bronchus. In a case in which the technique of the present disclosure is applied to an operative field in the tubular organ, for example, the marker M is provided in a base end part of a soft endoscope to be inserted into the tubular organ.

In each of the above-described embodiments, the medical device including the insertion part may be the trocar 17, in addition to the endoscope 13 and the medical probe (the ultrasound probe 14 or the OCT probe).

The following technical matters described in the following supplementary notes can be understood from the above description.

### Supplementary note 1

A medical support device comprising: a processor, in which the processor is configured to: acquire an intracorporeal image that is captured by an intracorporeal camera that images an inside of a body of a subject, the intracorporeal image including, within an imaging range, an organ punctured by a puncture needle, an insertion part of a medical device inserted into the body, and a marker that is provided at the insertion part and is image-recognizable; derive position/posture information including at least one of a position or a posture of the insertion part based on the marker; and execute display control of displaying a puncture path of the puncture needle in a superimposed form at a position specified in the intracorporeal image based on the position/posture information.

### Supplementary note 2

The medical support device according to supplementary note 1, in which the processor is configured to, based on two intracorporeal images that are captured from at least two different viewpoints and are the intracorporeal images in a state in which an operation of causing a change in an interior wall inside the body is performed from an outside of the body and the puncture path is displayed in a superimposed form, determine whether or not a change position at which the interior wall is changed and the puncture path intersect with each other in a three-dimensional space inside the body, and notify of a determination result.

### Supplementary note 3

The medical support device according to supplementary note 2, in which, in a case in which the change position at which the change occurs and the puncture path displayed in a superimposed form intersect with each other two-dimensionally in both of the two intracorporeal images, it is determined that the change position and the puncture path intersect with each other in the three-dimensional space.

### Supplementary note 4

The medical support device according to supplementary note 2 or 3, in which the processor is configured to, in a case in which the operation is performed a plurality of times, display a history of the change position of each of the plurality of times of the operations in a superimposed form on the intracorporeal image.

### Supplementary note 5

The medical support device according to supplementary note 4, in which the processor is configured to display the history of the change position by reflecting a movement amount of the viewpoint of the intracorporeal camera in a case in which the viewpoint is moved among the plurality of times of the operations.

### Supplementary note 6

The medical support device according to any one of supplementary notes 2 to 5, in which the operation is a pressing operation of pressing a body surface of the subject from the outside of the body, and the change position is a protrusion portion in which the interior wall protrudes in a body internal direction via the pressing operation.

### Supplementary note 7

The medical support device according to supplementary note 1, in which the processor is configured to derive an intersection point in a three-dimensional space between an interior wall inside the body and the puncture path based on depth information of the inside of the body indicating a distribution of a depth that is a distance in a depth direction from a viewpoint of the intracorporeal camera.

### Supplementary note 8

The medical support device according to supplementary note 7, in which a camera configured to estimate the depth is used as the intracorporeal camera, and the processor is configured to acquire the depth information.

### Supplementary note 9

The medical support device according to supplementary note 7, in which the processor is configured to acquire the depth information based on two intracorporeal images captured from different viewpoints.

### Supplementary note 10

The medical support device according to supplementary note 7, in which the processor is configured to acquire the depth information by using a machine learning model that receives input of the intracorporeal image and outputs the depth information.

### Supplementary note 11

The medical support device according to any one of supplementary notes 7 to 10, in which the processor is configured to, in a case in which the intracorporeal image in a state in which an operation of causing a change in an interior wall of a body cavity of the subject is performed from an outside of the body is acquired, determine whether or not a change position at which the interior wall is changed and the puncture path intersect with each other in the three-dimensional space inside the body based on the depth information, and notify of a determination result.

### Supplementary note 12

The medical support device according to any one of supplementary notes 1 to 11, in which the medical device is a medical probe configured to observe an internal structure of the organ.

### Supplementary note 13

The medical support device according to supplementary note 12, in which the medical probe is an ultrasound probe.

### Supplementary note 14

The medical support device according to supplementary note 12 or 13, in which the medical probe includes a guide groove provided at the insertion part and engaging with the puncture needle to guide insertion of the puncture needle to a target position inside the organ, and the processor is configured to specify the position in the intracorporeal image at which the puncture path is superimposed, based on a relative positional relationship between the marker and the guide groove.

### Supplementary note 15

The medical support device according to any one of supplementary notes 12 to 14, in which the processor is configured to specify the position in the intracorporeal image at which the puncture path is superimposed, based on a target position inside the organ specified in an internal image of the organ acquired by the medical probe, and a correlation between a coordinate system of the internal image and a coordinate system of the intracorporeal image, the correlation being derived based on the position/posture information.

### Supplementary note 16

The medical support device according to any one of supplementary notes 12 to 15, in which the processor is configured to, in a case in which the puncture path is set by a preoperative simulation in a three-dimensional image of the organ acquired in advance before a surgical operation, specify the position in the intracorporeal image at which the puncture path is superimposed, by using a correlation between a coordinate system of an internal image of the organ acquired by the medical probe and a coordinate system of the three-dimensional image, and a correlation between the coordinate system of the internal image and a coordinate system of the intracorporeal image, the correlation being derived based on the position/posture information.

### Supplementary note 17

The medical support device according to any one of supplementary notes 1 to 16, in which the processor is configured to change display of a part of the puncture path.

### Supplementary note 18

The medical support device according to supplementary note 17, in which the part of the puncture path is a part on an interior wall side inside the body, and the processor is configured to highlight the part on the interior wall side.

### Supplementary note 19

The medical support device according to supplementary note 17 or 18, in which the part of the puncture path is a part on an organ side, and the processor is configured to make visibility lower in the part on the organ side than in a part on an interior wall side.

### Supplementary Note 20

The medical support device according to any one of supplementary notes 17 to 19, in which the processor is configured to detect an insertion length of the insertion part of the medical device, and decide a range in which the display is changed, based on the detected insertion length.

### Supplementary Note 21

The medical support device according to any one of supplementary notes 17 to 20, in which the processor is configured to decide a range in which the display is changed, based on designation of a user.

### Supplementary Note 22

The medical support device according to any one of supplementary notes 1 to 21, in which the processor is configured to display a plurality of different puncture paths.

### Supplementary Note 23

An operation method of a medical support device including a processor, the operation method comprising: via the processor, acquiring an intracorporeal image that is captured by an intracorporeal camera that images an inside of a body of a subject, the intracorporeal image including, within an imaging range, an organ punctured by a puncture needle, an insertion part of a medical device inserted into the body, and a marker that is provided at the insertion part and is image-recognizable; deriving position/posture information including at least one of a position or a posture of the insertion part based on the marker; and executing display control of displaying a puncture path of the puncture needle in a superimposed form at a position specified in the intracorporeal image based on the position/posture information.

### Supplementary Note 24

An operation program of a medical support device, the operation program causing a computer to function as the medical support device and causing the computer to execute: a step of acquiring an intracorporeal image that is captured by an intracorporeal camera that images an inside of a body of a subject, the intracorporeal image including, within an imaging range, an organ punctured by a puncture needle, an insertion part of a medical device inserted into the body, and a marker that is provided at the insertion part and is image-recognizable; a step of deriving position/posture information including at least one of a position or a posture of the insertion part based on the marker; and a step of executing display control of displaying a puncture path of the puncture needle in a superimposed form at a position specified in the intracorporeal image based on the position/posture information.

### Supplementary Note 25

A medical support system comprising: a medical device that includes an insertion part to be inserted into an inside of a body of a subject and has a marker that is provided at the insertion part and is image-recognizable; an intracorporeal camera that images an intracorporeal image including, within an imaging range, the insertion part, the marker, and an organ punctured by a puncture needle; and a medical support device including a processor, in which the processor is configured to: acquire the intracorporeal image; derive position/posture information including at least one of a position or a posture of the insertion part based on the marker; and execute display control of displaying a puncture path of the puncture needle in a superimposed form at a position specified in the intracorporeal image based on the position/posture information.

The following technical matters described in the following second supplementary notes can be understood from the above description. The technique described in the second supplementary notes relates to a puncture method performed by the medical staff ST while visually recognizing the operative field image 21 of the first viewpoint VP1 and the operative field image 21 of the second viewpoint VP2 described in the second embodiment.

### Second supplementary note 1

A puncture method including: performing an operation of causing a change in an interior wall of an inside of a body of a subject from an outside of the body while visually recognizing an intracorporeal image that is captured by an intracorporeal camera that images the inside of the body the intracorporeal image including, within an imaging range, an organ punctured by a puncture needle, an insertion part of a medical device inserted into the body, and a marker that is provided at the insertion part and is image-recognizable, and in which a puncture path of the puncture needle is displayed in a superimposed form on a position specified based on position/posture information including at least one of a position or a posture of the insertion part derived based on the marker; determining whether or not a change position in which the interior wall is changed and the puncture path intersect with each other in a three-dimensional space inside the body based on a first intracorporeal image captured from a first viewpoint and a second intracorporeal image captured from a second viewpoint different from the first viewpoint in a state in which the operation is performed; and puncturing an intersection point between the change position and the puncture path by the puncture needle in a case in which it is determined in a determination result that the change position and the puncture path intersect with each other.

### Second supplementary note 2

The puncture method according to second supplementary note 1, in which the operation includes two operations of a first operation and a second operation performed at different timings on a same position, a first determination of visually recognizing the first intracorporeal image in a state in which the first operation is performed, to determine whether or not the change position in the first intracorporeal image and the puncture path intersect with each other is performed, a second determination of visually recognizing the second intracorporeal image in a state in which the second operation is performed, to determine whether or not the change position in the second intracorporeal image and the puncture path intersect with each other is performed, and it is determined that the change position and the puncture path intersect with each other in the three-dimensional space in a case in which it is determined that it is determined in both the first determination and the second determination that the change position and the puncture path intersect with each other.

### Second supplementary note 3

The puncture method according to second supplementary note 1, in which a first determination of visually recognizing the first intracorporeal image in a state in which the operation is performed, to determine whether or not the change position in the first intracorporeal image and the puncture path intersect with each other, and a second determination of visually recognizing the second intracorporeal image in a state in which the operation is continued, to determine whether or not the change position in the second intracorporeal image and the puncture path intersect with each other are performed, and it is determined that the change position and the puncture path intersect with each other in the three-dimensional space in a case in which it is determined that it is determined in both the first determination and the second determination that the change position and the puncture path intersect with each other.

In addition, in the above-described embodiments, for example, as the hardware structure of the processor 41 that executes various types of processing such as the image acquisition, the position/posture information derivation, the image combining, and the display control, various processors shown below can be used. The various processors include, in addition to a CPU that is a general-purpose processor that executes software (program) to function as various processing units, a programmable logic device (PLD) of which a circuit configuration can be changed after manufacturing, such as a field-programmable gate array (FPGA), and a dedicated electric circuit that is a processor having a circuit configuration dedicatedly designed for executing specific processing, such as an application specific integrated circuit (ASIC).

Various types of processing described above may be executed by one of the various processors or may be executed by a combination of two or more processors (for example, a combination of a plurality of FPGAs or a CPU and an FPGA) of the same type or different types. A plurality of processing units may be configured by one processor. As an example in which the plurality of processing units are configured by one processor, there is a form in which a processor that realizes all functions of a system including the plurality of processing units by using one integrated circuit (IC) chip is used, such as a system on a chip (SOC).

In this way, as the hardware structure, the various processing units are constituted by one or more of the various processors described above.

Further, the hardware structure of these various processors is, more specifically, an electric circuit (circuitry) in which circuit elements such as semiconductor elements are combined.

In addition to the operation program of the medical support device, the technique of the present disclosure extends to a computer readable storage medium (USB memory or digital versatile disc (DVD)-read only memory (ROM), or the like) that stores the operation program of the medical support device in a non-transitory manner.

The above-described contents and the above-shown contents are detailed descriptions for parts according to the technique of the present disclosure, and are merely examples of the technique of the present disclosure. For example, the above descriptions of the configurations, the functions, the actions, and the effects are the descriptions of examples of the configurations, the functions, the actions, and the effects of the parts according to the technique of the present disclosure. Accordingly, it is needless to say that unnecessary parts may be deleted, new elements may be added, or replacements may be made with respect to the above-described contents and the above-shown contents within a range that does not deviate from the gist of the technique of the present disclosure. In addition, in order to avoid complications and facilitate understanding of the parts according to the technique of the present disclosure, in the above-described contents and the above-shown contents, the description of common technical knowledge and the like that do not particularly require description for enabling the implementation of the technique of the present disclosure are omitted.

In the present specification, "A and/or B" is synonymous with "at least one of A or B". That is, "A and/or B" means that it may be only A, only B, or a combination of A and B. In addition, in the present specification, in a case in which three or more matters are associated and expressed by "and/or", the same concept as "A and/or B" is applied.

All documents, patent applications, and technical standards described in the present specification are incorporated into the present specification by reference to the same extent as in a case in which the individual documents, patent applications, and technical standards are specifically and individually stated to be described by reference.

## Claims

1. A medical support device (11) comprising:
a processor (41),
wherein the processor is configured to:
acquire an intracorporeal image (21) that is captured by an intracorporeal camera (13B) that images an inside of a body of a subject, the intracorporeal image including, within an imaging range, an organ punctured by a puncture needle (18), an insertion part (14A) of a medical device (14) inserted into the body, and a marker (M) that is provided at the insertion part and is image-recognizable;
derive position/posture information including at least one of a position or a posture of the insertion part (14A) based on the marker; and
execute display control of displaying a puncture path of the puncture needle (18) in a superimposed form at a position specified in the intracorporeal image (21) based on the position/posture information.

2. The medical support device according to claim 1,
wherein the processor (41) is configured to, based on two intracorporeal images that are captured from at least two different viewpoints and are the intracorporeal images in a state in which an operation of causing a change in an interior wall (W) inside the body is performed from an outside of the body and the puncture path is displayed in a superimposed form, determine whether or not a change position at which the interior wall is changed and the puncture path intersect with each other in a three-dimensional space inside the body, and notify of a determination result.

3. The medical support device according to claim 2,
wherein, in a case in which the change position at which the change occurs and the puncture path displayed in a superimposed form intersect with each other two-dimensionally in both of the two intracorporeal images, it is determined that the change position and the puncture path intersect with each other in the three-dimensional space.

4. The medical support device according to claim 2,
wherein the processor (41) is configured to,
in a case in which the operation is performed a plurality of times,
display a history of the change position of each of the plurality of times of the operations in a superimposed form on the intracorporeal image.

5. The medical support device according to claim 4,
wherein the processor (41) is configured to display the history of the change position by reflecting a movement amount of the viewpoint of the intracorporeal camera in a case in which the viewpoint is moved among the plurality of times of the operations.

6. The medical support device according to claim 2,
wherein the operation is a pressing operation of pressing a body surface of the subject from the outside of the body, and
the change position is a protrusion portion in which the interior wall protrudes in a body internal direction via the pressing operation.

7. The medical support device according to claim 1,
wherein the processor (41) is configured to derive an intersection point in a three-dimensional space between an interior wall inside the body and the puncture path based on depth information of the inside of the body indicating a distribution of a depth that is a distance in a depth direction from a viewpoint of the intracorporeal camera.

8. The medical support device according to claim 7,
wherein a camera configured to estimate the depth is used as the intracorporeal camera, and
the processor is configured to acquire the depth information.

9. The medical support device according to claim 7,
wherein the processor (41) is configured to acquire the depth information based on two intracorporeal images captured from different viewpoints.

10. The medical support device according to claim 7,
wherein the processor is configured to acquire the depth information by using a machine learning model that receives input of the intracorporeal image and outputs the depth information.

11. The medical support device according to claim 7,
wherein the processor is configured to,
in a case in which the intracorporeal image in a state in which an operation of causing a change in an interior wall of a body cavity of the subject is performed from an outside of the body is acquired,
determine whether or not a change position at which the interior wall is changed and the puncture path intersect with each other in the three-dimensional space inside the body based on the depth information, and notify of a determination result.

12. The medical support device according to claim 1,
wherein the medical device is a medical probe configured to observe an internal structure of the organ.

13. The medical support device according to claim 12,
wherein the medical probe is an ultrasound probe.

14. The medical support device according to claim 12,
wherein the medical probe includes a guide groove provided at the insertion part and engaging with the puncture needle to guide insertion of the puncture needle to a target position inside the organ, and
the processor is configured to specify the position in the intracorporeal image at which the puncture path is superimposed, based on a relative positional relationship between the marker and the guide groove.

15. The medical support device according to claim 12,
wherein the processor is configured to specify the position in the intracorporeal image (21) at which the puncture path is superimposed, based on a target position inside the organ specified in an internal image of the organ acquired by the medical probe, and a correlation between a coordinate system of the internal image and a coordinate system of the intracorporeal image, the correlation being derived based on the position/posture information.

16. The medical support device according to claim 12,
wherein the processor (41) is configured to,
in a case in which the puncture path is set by a preoperative simulation in a three-dimensional image of the organ acquired in advance before a surgical operation,
specify the position in the intracorporeal image at which the puncture path is superimposed, by using a correlation between a coordinate system of an internal image of the organ acquired by the medical probe and a coordinate system of the three-dimensional image, and a correlation between the coordinate system of the internal image and a coordinate system of the intracorporeal image, the correlation being derived based on the position/posture information.

17. The medical support device according to claim 1,
wherein the processor (41) is configured to change display of a part of the puncture path.

18. The medical support device according to claim 17,
wherein the part of the puncture path is a part on an interior wall side inside the body, and
the processor is configured to highlight the part on the interior wall side.

19. The medical support device according to claim 17,
wherein the part of the puncture path is a part on an organ side, and
the processor (41) is configured to make visibility lower in the part on the organ side than in a part on an interior wall side.

20. The medical support device according to claim 17,
wherein the processor (41) is configured to detect an insertion length of the insertion part of the medical device, and decide a range in which the display is changed, based on the detected insertion length.

21. The medical support device according to claim 17,
wherein the processor (41) is configured to decide a range in which the display is changed, based on designation of a user.

22. The medical support device according to claim 1,
wherein the processor (41) is configured to display a plurality of different puncture paths.

23. An operation method of a medical support device (11) including a processor (41), the operation method comprising:
via the processor,
acquiring an intracorporeal image (21) that is captured by an intracorporeal camera (13B) that images an inside of a body of a subject, the intracorporeal image (21) including, within an imaging range, an organ punctured by a puncture needle (18), an insertion part (14A) of a medical device (14) inserted into the body, and a marker (M) that is provided at the insertion part and is image-recognizable;
deriving position/posture information including at least one of a position or a posture of the insertion part (14A) based on the marker; and
executing display control of displaying a puncture path of the puncture needle (18) in a superimposed form at a position specified in the intracorporeal image (21) based on the position/posture information.

24. A computer-readable storage medium storing a operation program of a medical support device, the operation program causing a computer to function as the medical support device and causing the computer to execute:
a process of acquiring an intracorporeal image (21) that is captured by an intracorporeal camera (13B) that images an inside of a body of a subject, the intracorporeal image including, within an imaging range, an organ punctured by a puncture needle (18), an insertion part (14A) of a medical device (14) inserted into the body, and a marker (M) that is provided at the insertion part and is image-recognizable;
a process of deriving position/posture information including at least one of a position or a posture of the insertion part (14A) based on the marker; and
a process of executing display control of displaying a puncture path of the puncture needle in a superimposed form at a position specified in the intracorporeal image (21) based on the position/posture information.

25. A medical support system (10) comprising:
a medical device (14) that includes an insertion part (14A) to be inserted into an inside of a body of a subject and has a marker (M) that is provided at the insertion part and is image-recognizable;
an intracorporeal camera (13B) that images an intracorporeal image (21) including, within an imaging range, the insertion part, the marker, and an organ punctured by a puncture needle; and
a medical support device including a processor (41),
wherein the processor is configured to:
acquire the intracorporeal image (21);
derive position/posture information including at least one of a position or a posture of the insertion part based on the marker; and
execute display control of displaying a puncture path of the puncture needle in a superimposed form at a position specified in the intracorporeal image (21) based on the position/posture information.
